(19) Europäisches Patentamt
European Patent Office
Office européen des brevets

(11) **EP 3 555 288 B1**

(12) **EUROPEAN PATENT SPECIFICATION**

(45) Date of publication and mention
of the grant of the patent:
**03.02.2021 Bulletin 2021/05**

(21) Application number: **17833043.7**

(22) Date of filing: **19.12.2017**

(51) Int Cl.:
**C12N 15/10** (2006.01)

(86) International application number:
**PCT/US2017/067382**

(87) International publication number:
**WO 2018/118968 (28.06.2018 Gazette 2018/26)**

(54) **EMULSION-BASED SCREENING METHODS**

EMULSIONSBASIERTE SCREENING-VERFAHREN

MÉTHODES DE CRIBLAGE FAISANT APPEL À UNE ÉMULSION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **19.12.2016 US 201662436235 P**

(43) Date of publication of application:
**23.10.2019 Bulletin 2019/43**

(73) Proprietor: **Editas Medicine, Inc.
Cambridge, MA 02141 (US)**

(72) Inventor: **STEINBERG, Barrett Ethan
Somerville, MA 02144 (US)**

(74) Representative: **Forresters IP LLP
Skygarden
Erika-Mann-Straße 11
80636 München (DE)**

(56) References cited:
**WO-A2-2008/103900**

- **ADAMSON BRITT ET AL: "A Multiplexed
Single-Cell CRISPR Screening Platform Enables
Systematic Dissection of the Unfolded Protein
Response", CELL, CELL PRESS, vol. 167, no. 7,
15 December 2016 (2016-12-15), page 1867,
XP029850719, ISSN: 0092-8674, DOI:
10.1016/J.CELL.2016.11.048**

- **DOI NOBUHIDE ET AL: "In vitro selection of
restriction endonucleases by in vitro
compartmentalization", NUCLEIC ACIDS
RESEARCH, INFORMATION RETRIEVAL LTD,
GB, vol. 32, no. 12, 1 January 2004 (2004-01-01),
pages e95.1-e95.8, XP002485451, ISSN:
0305-1048, DOI: 10.1093/NAR/GNH096**

- **TAKEUCHI R ET AL: "Redesign of extensive
protein-DNA interfaces of meganucleases using
iterative cycles of in vitro compartmentalization",
PROCEEDINGS NATIONAL ACADEMY OF
SCIENCES PNAS, NATIONAL ACADEMY OF
SCIENCES, US, vol. 111, no. 11, 1 March 2014
(2014-03-01), pages 4061-4066, XP002757991,
ISSN: 0027-8424, DOI: 10.1073/PNAS.1321030111
[retrieved on 2014-03-03]**

- **Ryo Takeuchi ET AL: "Engineering of Customized
Meganucleases via In Vitro
Compartmentalization and In Cellulo
Optimization" In: "Methods in Molecular
Biology", 3 November 2014 (2014-11-03), Humana
Press, Inc., US, XP055462304, ISSN: 1064-3745
vol. 1239, pages 105-132, DOI:
10.1007/978-1-4939-1862-1_6, the whole
document**

- **CONG LE ET AL: "Multiplex Genome Engineering
Using CRISPR/Cas Systems", SCIENCE
(WASHINGTON D C), vol. 339, no. 6121, February
2013 (2013-02), pages 819-823, XP002779503,
ISSN: 0036-8075**

**Description**

**BACKGROUND**

Nucleases such as zinc finger nucleases (ZFNs), transcription activator-like effector nucleases (TALENs), and clustered regularly-interspersed short palindromic repeat (CRISPR)-associated nucleases have become increasingly used because of their ability to be targeted to particular DNA sequences. The value of nucleases such as these as a tool for the treatment of inherited diseases is widely recognized. For example, the U.S. Food and Drug Administration (FDA) held a Science Board Meeting on November 15, 2016 addressing the use of such systems and potential regulatory considerations raised by them. In that meeting, the FDA noted that while Cas9/guide RNA (gRNA) ribonucleoprotein (RNP) complexes may be customized to generate precise edits at a locus of interest, the complexes may also interact with, and cut at, other "off-target" loci. The potential for off-target cuts ("off-targets"), in turn, raises at least a potential regulatory consideration with respect to the approval of therapeutics utilizing these nucleases.

**SUMMARY**

The present disclosure provides, among other things, methods and systems for characterization of nuclease activity using compartmentalized in vitro transcription and translation, such as an emulsified in vitro transcription and translation system.

In one aspect, the disclosure provides methods comprising steps of: (a) emulsifying a library comprising a plurality of variant nucleic acid templates to form droplets, wherein each variant nucleic acid template comprises: (i) a first nucleotide sequence encoding a nuclease operably linked to a first promoter; and (ii) a second nucleotide sequence comprising a target site for the nuclease; and wherein each of the plurality of the droplets comprises a unique variant nucleic acid template; (b) expressing the nuclease in the plurality of the droplets; (c) subjecting the plurality of the droplets to conditions favorable for nuclease cleavage of the target site to produce a population of cleaved nucleic acid templates, each cleaved nucleic acid template comprising the first nucleotide sequence and a predetermined cleaved end; and (d) ligating the cleaved nucleic acid templates with at least one oligonucleotide capture probe specific for the predetermined cleaved end to produce a plurality of ligation products.

In some embodiments, the method further comprises disrupting the droplets to obtain a mixture comprising cleaved nucleic acid templates, prior to the ligating step.

In some embodiments, the method further comprises detecting at least one ligation product. In some embodiments, the method further comprises amplifying at least one ligation product. In some embodiments, the method further comprises sequencing at least one ligation product.

In some embodiments, the nuclease is an RNA-guided nuclease. In some embodiments, each variant nucleic acid template further comprises a third nucleotide sequence encoding a guide RNA. In some embodiments, the third nucleotide sequence is operably linked to the first promoter. In some embodiments, the third nucleotide sequence is operably linked to a second promoter.

In some embodiments, each variant nucleic acid template further comprises a fourth nucleotide sequence adjacent the target site, the fourth nucleotide sequence comprising a protospacer adjacent motif (PAM).

In some embodiments, each variant nucleic acid template comprises a first nucleotide sequence encoding a variant of a nuclease operably linked to the first promoter.

In some embodiments, each variant nucleic acid template further comprises a third nucleotide sequence encoding a variant of a guide RNA.

In some embodiments, each variant nucleic acid template further comprises a third nucleotide sequence adjacent the target site, the third nucleotide sequence comprising a variant of a PAM

In some embodiments, each variant nucleic acid template comprises a second nucleotide sequence comprising a candidate target site for the nuclease.

In some embodiments, each variant nucleic acid template comprises (i) a target site 5' to the first nucleotide sequence and a barcode sequence situated between the target site and the first nucleotide sequence or (ii) a target site 5' to the first nucleotide sequence and a barcode sequence 3' to the first nucleotide sequence.

In another aspect, the disclosure provides methods comprising steps of: (a) emulsifying a library comprising a plurality of variant nucleic acid templates to form droplets, wherein each variant nucleic acid template comprises: (i) a first nucleotide sequence encoding a nuclease operably linked to a first promoter; and (ii) a second nucleotide sequence comprising a target site for the nuclease; and wherein each of the plurality of the droplets comprises a unique variant nucleic acid template; (b) expressing the nuclease in the plurality of the droplets; (c) subjecting the plurality of the droplets to conditions favorable for nuclease cleavage of the target site to produce a population of cleaved nucleic acid templates, each cleaved nucleic acid template comprising the first nucleotide sequence and a predetermined cleaved end; and (d) detecting at least a portion of the cleaved nucleic acid templates comprising a blunt end.

In another aspect, the disclosure provides methods comprising steps of: (a) emulsifying a library comprising a plurality of nucleic acid templates to form droplets, wherein each nucleic acid template comprises: (i) a first nucleotide sequence encoding a variant of a nuclease operably linked to a first promoter; and (ii) a second nucleotide sequence comprising a target site for the nuclease; and wherein each of a plurality of the droplets comprises a unique nucleic acid template; (b) expressing the nuclease variants in the plurality of the droplets; (c) subjecting the plurality of the droplets to conditions favorable for nuclease cleavage of the target site to produce a population of cleaved nucleic acid templates, each cleaved nucleic acid template comprising the first nucleotide sequence and a predetermined cleaved end; (d) ligating the cleaved nucleic acid templates with an oligonucleotide capture probe specific for the predetermined cleaved end to produce a plurality of ligation products; and (e) identifying at least one first nucleotide sequence encoding a nuclease variant in at least one ligation product.

In another aspect, the disclosure provides methods comprising steps of: (a) emulsifying a library comprising a plurality of nucleic acid templates to form droplets, wherein each nucleic acid template comprises: (i) a first nucleotide sequence encoding an RNA-guided nuclease operably linked to a first promoter; (ii) a second nucleotide sequence encoding a variant of a guide RNA operably linked to a second promoter; and (iii) a third nucleotide sequence comprising a target site for the guide RNA; and wherein each of a plurality of the droplets comprises a unique nucleic acid template; (b) expressing the nuclease and guide RNA variants in the plurality of the droplets to form nuclease/guide RNA variant complexes; (c) subjecting the plurality of the droplets to conditions favorable for cleavage of the target site by a plurality of nuclease/guide RNA complexes to produce a population of cleaved nucleic acid templates, each cleaved nucleic acid template comprising the second nucleotide sequence and a predetermined cleaved end; (d) ligating the cleaved nucleic acid templates with an oligonucleotide capture probe specific for the predetermined cleaved end to produce a plurality of ligation products; and (e) identifying at least one second nucleotide sequence encoding a guide RNA variant in at least one ligation product.

In another aspect, the disclosure provides methods comprising steps of: (a) emulsifying a library comprising a plurality of nucleic acid templates to form droplets, wherein each nucleic acid template comprises: (i) a first nucleotide sequence encoding a nuclease operably linked to a first promoter; (ii) a second nucleotide sequence comprising a target site for the nuclease; and (iii) a third nucleotide sequence adjacent the target site, the third nucleotide sequence comprising a variant of a PAM; and wherein each of a plurality of the droplets comprises a unique nucleic acid template; (b) expressing the nuclease in the plurality of the droplets; (c) subjecting the plurality of the droplets to conditions favorable for nuclease cleavage of the target site to produce a population of cleaved nucleic acid templates, each cleaved nucleic acid template comprising the third nucleotide sequence and a predetermined cleaved end; (d) ligating the cleaved nucleic acid templates with an oligonucleotide capture probe specific for the predetermined cleaved end to produce a plurality of ligation products; and (e) identifying at least one third nucleotide sequence comprising a PAM variant in at least one ligation product.

In another aspect, the disclosure provides methods comprising steps of: (a) emulsifying a library comprising a plurality of nucleic acid templates to form droplets, wherein each nucleic acid template comprises: (i) a first nucleotide sequence encoding a nuclease operably linked to a first promoter; and (ii) a second nucleotide sequence comprising a candidate target site for the nuclease and comprising a unique molecular identifier; and wherein each of a plurality of the droplets comprises a unique nucleic acid template; (b) expressing the nuclease in the plurality of the droplets; (c) subjecting the plurality of the droplets to conditions favorable for nuclease cleavage of the candidate target site to produce a population of cleaved nucleic acid templates, each cleaved nucleic acid template comprising a unique molecular identifier and a predetermined cleaved end; (d) ligating the cleaved nucleic acid templates with an oligonucleotide capture probe specific for the predetermined cleaved end to produce a plurality of ligation products; and (e) identifying at least one unique molecular identifier associated with a candidate target site in at least one ligation product.

In a further embodiment the present invention provides a method comprising steps of: (a) emulsifying a library comprising a plurality of variant nucleic acid templates to form droplets, wherein each variant nucleic acid template comprises: (i) a first nucleotide sequence encoding a nuclease operably linked to a first promoter; and (ii) a second nucleotide sequence comprising a target site for the nuclease; and (iii) a third nucleotide sequence encoding a guide RNA; wherein each of the plurality of the droplets comprises a unique variant nucleic acid template; (b) expressing the nuclease in the plurality of the droplets; (c) subjecting the plurality of the droplets to conditions favorable for nuclease cleavage of the target site to produce a population of cleaved nucleic acid templates, each cleaved nucleic acid template comprising the first nucleotide sequence and a predetermined cleaved end; (d) ligating the cleaved nucleic acid templates with a plurality of oligonucleotide capture probe each specific for a different predetermined cleaved end to produce a plurality of ligation products, and (e) further comprising detecting at least one ligation product.

In any of the aspect described herein, the predetermined cleaved end can comprise a 5' phosphate group. In some embodiments, the predetermined cleaved end is a blunt end. In some embodiments, the predetermined cleaved end is a cohesive end. In some embodiments, the cohesive end comprises a 3' overhang with a predetermined number of nucleotides. In some embodiments, the cohesive end comprises a 5' overhang with a predetermined number of nucleotides.

In any of the aspects described herein, the ligating step can comprise incubating with a T4 ligase. In some embodiments, the ligating step comprises incubating with E. coli ligase.

In any of the aspects described herein, step (d) can comprise ligating the cleaved nucleic acid templates with a plurality of oligonucleotide capture probes. In some embodiments, each oligonucleotide capture probe is specific for a different predetermined cleaved end. In some embodiments, each of the oligonucleotide capture probes comprises a unique detectable label associated with a predetermined cleaved end. In some embodiments, the unique detectable label comprises a barcode sequence. In some embodiments, the unique detectable label comprises a fluorescent marker. In some embodiments, each of the oligonucleotide capture probes comprises a randomized barcode sequence not associated with a predetermined cleaved end. In some embodiments, the method further comprises detecting a randomized barcode sequence in at least a plurality of the ligation products. In some embodiments, the method further comprises a step of analyzing, for a plurality of variant nucleic acid templates, the distribution of detected randomized barcode sequences present in the plurality of ligation products.

In any of the aspects described herein, the step of emulsifying can comprise forming an aqueous phase comprising the library of variant nucleic acid templates. In some embodiments, the emulsifying step further comprises adding the aqueous phase to a mixture comprising oil and surfactant to form a water-in-oil emulsion.

In any of the aspects described herein, the library can comprise at least $10^4$, at least $10^5$, at least $10^6$, at least $10^7$, at least $10^8$, or at least $10^9$ variant nucleic acid templates. In some embodiments, the library comprises about $10^4$, $10^5$, $10^6$, $10^7$, $10^8$, or $10^9$ variant nucleic acid templates. In some embodiments, the library comprises about $10^4$ to about $10^9$, or about $10^5$ to about $10^8$ variant nucleic acid templates variant nucleic acid templates.

In another aspect, the disclosure provides a library comprising a plurality of variant nucleic acid templates encoding variants of a nuclease described herein, wherein each variant nucleic acid template comprises: (i) a first nucleotide sequence comprising a detection sequence described herein and encoding a nuclease operably linked to a first promoter; and (ii) a second nucleotide sequence comprising a target site for the nuclease.

In another aspect, the disclosure provides a library comprising a plurality of variant nucleic acid templates encoding variants of a nuclease described herein, wherein each variant nucleic acid template comprises: (i) a first nucleotide sequence encoding a nuclease variant operably linked to a first promoter; and (ii) a second nucleotide sequence comprising a target site for the nuclease, wherein upon expression of the nuclease variants, one or more nuclease variants cleave one or more nucleic acid templates producing a population of detectable cleaved nucleic acid templates comprising the first nucleotide sequence, wherein at least a portion of the detectable cleaved nucleic acid templates comprise a blunt end.

In another aspect, the disclosure provides a library comprising a plurality of variant nucleic acid templates encoding variants of a nuclease described herein, wherein each variant nucleic acid template comprises: (i) a first nucleotide sequence comprising a detection sequence described herein and encoding a nuclease variant operably linked to a first promoter; and (ii) a second nucleotide sequence comprising a target site for the nuclease, wherein upon expression of the nuclease variants, one or more nuclease variants cleave one or more nucleic acid templates producing a population of cleaved nucleic acid templates, each comprising the first nucleotide sequence and a predetermined cleaved end (e.g., a cleaved end described herein) to which an oligonucleotide capture probe (e.g., a capture probe described herein) specific for the predetermined cleaved end can ligate.

In another aspect, the disclosure provides an emulsion comprising a plurality of droplets comprising a plurality of variant nucleic acid templates encoding variants of a nuclease described herein, wherein each droplet comprises a unique nucleic acid template comprising: (i) a first nucleotide sequence comprising a detection sequence described herein and encoding a nuclease variant operably linked to a first promoter; and (ii) a second nucleotide sequence comprising a target site for the nuclease.

In another aspect, the disclosure provides an emulsion comprising a plurality of droplets comprising a plurality of variant nucleic acid templates encoding variants of a nuclease described herein, wherein each droplet comprises a unique nucleic acid template comprising: (i) a first nucleotide sequence comprising a detection sequence described herein and encoding a nuclease variant operably linked to a first promoter; and (ii) a second nucleotide sequence comprising a target site for the nuclease, wherein upon expression of the nuclease variants, one or more nuclease variants cleave one or more nucleic acid templates producing a population of detectable cleaved nucleic acid templates comprising the first nucleotide sequence, wherein at least a portion of the detectable cleaved nucleic acid templates comprise a blunt end.

In another aspect, the disclosure provides an emulsion comprising a plurality of droplets comprising a plurality of variant nucleic acid templates encoding variants of a nuclease described herein, wherein each droplet comprises a unique nucleic acid template comprising: (i) a first nucleotide sequence comprising a detection sequence described herein and encoding a nuclease variant operably linked to a first promoter; and (ii) a second nucleotide sequence comprising a target site for the nuclease, wherein upon expression of the nuclease variants, one or more nuclease variants cleave one or more nucleic acid templates producing a population of cleaved nucleic acid templates, each comprising the first nucleotide sequence and a predetermined cleaved end (e.g., a predetermined cleaved end described herein) to which an oligonucleotide capture probe (e.g., a capture probe described herein) specific for the predetermined cleaved end can ligate.

In another aspect, the disclosure provides a composition comprising a plurality of cleaved variant nucleic acid templates and a plurality of uncleaved variant nucleic acid templates, wherein each uncleaved variant nucleic acid template comprises (i) a first nucleotide sequence encoding a nuclease variant described herein; (ii) a second nucleotide sequence comprising a target site for the nuclease, and (iii) a detection sequence described herein; and wherein each cleaved variant nucleic acid template comprises (i) a first nucleotide sequence encoding a nuclease variant described herein, (ii) an oligonucleotide capture probe described herein, wherein the oligonucleotide capture probe is ligated to the first nucleotide sequence by ligation to a predetermined cleaved end of the first nucleotide sequence, and (iii) a detection sequence described herein. In some embodiments, the cleaved and uncleaved variant nucleic acid templates are derived from or produced from a library of nucleic acid templates described herein.

In another aspect, the disclosure provides a library comprising a plurality of variant nucleic acid templates encoding variants of a guide RNA described herein, wherein each nucleic acid template comprises: (i) a first nucleotide sequence encoding a nuclease described herein (e.g., an RNA-guided nuclease described herein) operably linked to a first promoter; (ii) a second nucleotide sequence comprising a detection sequence described herein and encoding a variant of a guide RNA operably linked to a second promoter; and (iii) a third nucleotide sequence comprising a target site for the guide RNA. In some embodiments, the first and second promoters are the same promoter.

In another aspect, the disclosure provides a library comprising a plurality of variant nucleic acid templates encoding variants of a guide RNA described herein, wherein each variant nucleic acid template comprises: (i) a first nucleotide sequence encoding a nuclease described herein (e.g., an RNA-guided nuclease described herein) operably linked to a first promoter; (ii) a second nucleotide sequence comprising a detection sequence and encoding a variant of a guide RNA operably linked to a second promoter; and (iii) a third nucleotide sequence comprising a target site for the guide RNA, wherein upon expression of the nuclease and the guide RNA variants, the nuclease and one or more guide RNA variants form a nuclease/guide RNA variant complex that cleaves one or more nucleic acid templates producing a population of detectable cleaved nucleic acid templates comprising the second nucleotide sequence, wherein at least a portion of the detectable cleaved nucleic acid templates comprise a blunt end.

In another aspect, the disclosure provides a library comprising a plurality of variant nucleic acid templates encoding variants of a guide RNA described herein, wherein each variant nucleic acid template comprises: (i) a first nucleotide sequence encoding a nuclease described herein (e.g., an RNA-guided nuclease described herein) operably linked to a first promoter; (ii) a second nucleotide sequence comprising a detection sequence described herein and encoding a variant of a guide RNA operably linked to a second promoter; and (iii) a third nucleotide sequence comprising a target site for the guide RNA, wherein upon expression of the nuclease and the guide RNA variants, the nuclease and one or more guide RNA variants form a nuclease/guide RNA variant complex that cleaves one or more nucleic acid templates producing a population of cleaved nucleic acid templates, each comprising the second nucleotide sequence and a predetermined cleaved end to which an oligonucleotide capture probe (e.g., an oligonucleotide capture probe described herein) specific for the predetermined cleaved end can ligate.

In another aspect, the disclosure provides an emulsion comprising a plurality of droplets comprising a plurality of variant nucleic acid templates encoding variants of a guide RNA described herein, wherein each of the droplets comprises a unique variant nucleic acid template comprising: (i) a first nucleotide sequence encoding a nuclease described herein (e.g., an RNA-guided nuclease described herein) operably linked to a first promoter; (ii) a second nucleotide sequence comprising a detection sequence described herein and encoding a variant of a guide RNA operably linked to a second promoter; and (iii) a third nucleotide sequence comprising a target site for the guide RNA.

In another aspect, the disclosure provides an emulsion comprising a plurality of droplets comprising a plurality of variant nucleic acid templates encoding variants of a guide RNA described herein, wherein each of the droplets comprises a unique variant nucleic acid template comprising: (i) a first nucleotide sequence encoding a nuclease described herein (e.g., an RNA-guided nuclease described herein) operably linked to a first promoter; (ii) a second nucleotide sequence comprising a detection sequence described herein and encoding a variant of a guide RNA operably linked to a second promoter; and (iii) a third nucleotide sequence comprising a target site for the guide RNA, wherein upon expression of the nuclease and the guide RNA variants, the nuclease and one or more guide RNA variants form a nuclease/guide RNA variant complex that cleaves one or more nucleic acid templates producing a population of detectable cleaved nucleic acid templates comprising the second nucleotide sequence, wherein at least a portion of the detectable cleaved nucleic acid templates comprise a blunt end.

In another aspect, the disclosure provides an emulsion comprising a plurality of droplets comprising a plurality of variant nucleic acid templates encoding variants of a guide RNA described herein, wherein each of the droplets comprises a unique variant nucleic acid template comprising: (i) a first nucleotide sequence encoding a nuclease described herein (e.g., an RNA-guided nuclease described herein) operably linked to a first promoter; (ii) a second nucleotide sequence comprising a detection sequence described herein and encoding a variant of a guide RNA operably linked to a second promoter; and (iii) a third nucleotide sequence comprising a target site for the guide RNA, wherein upon expression of the nuclease and the guide RNA variants, the nuclease and one or more guide RNA variants form a nuclease/guide RNA variant complex that cleaves one or more nucleic acid templates producing a population of cleaved nucleic acid templates, each comprising the second nucleotide sequence and a predetermined cleaved end to which an oligonucleotide capture probe (e.g., an oligonucleotide capture probe described herein) specific for the predetermined cleaved end can ligate.

In another aspect, the disclosure provides a composition comprising a plurality of cleaved variant nucleic acid templates and a plurality of uncleaved variant nucleic acid templates, wherein each uncleaved variant nucleic acid template comprises (i) a first nucleotide sequence encoding a nuclease described herein (e.g., an RNA-guided nuclease described herein) operably linked to a first promoter; (ii) a second nucleotide sequence comprising a detection sequence described herein and encoding a variant of a guide RNA described herein operably linked to a second promoter; and (iii) a third nucleotide sequence comprising a target site for the guide RNA, and wherein each cleaved variant nucleic acid template comprises (i) a first nucleotide sequence encoding a guide RNA variant described herein, (ii) an oligonucleotide capture probe, wherein the oligonucleotide capture probe is ligated to the first nucleotide sequence by ligation to a predetermined cleaved end of the first nucleotide sequence, and (iii) a detection sequence. In some embodiments, the cleaved and uncleaved variant nucleic acid templates are derived from or produced from a library of nucleic acid templates described herein.

In another aspect, the disclosure provides a library comprising a plurality of variant nucleic acid templates comprising candidate target sites for a nuclease described herein, wherein each variant nucleic acid template comprises: (i) a first nucleotide sequence encoding a nuclease operably linked to a first promoter; and (ii) a second nucleotide sequence comprising a detection sequence and a candidate target site for the nuclease.

In another aspect, the disclosure provides a library comprising a plurality of variant nucleic acid templates comprising candidate target sites for a nuclease described herein, wherein each variant nucleic acid template comprises: (i) a first nucleotide sequence encoding a nuclease operably linked to a first promoter; and (ii) a second nucleotide sequence comprising a detection sequence described herein and a candidate target site for the nuclease, wherein upon expression of the nuclease, the nuclease cleaves one or more nucleic acid templates producing a population of detectable cleaved nucleic acid templates comprising the detection sequence, wherein at least a portion of the detectable cleaved nucleic acid templates comprise a blunt end.

In another aspect, the disclosure provides a library comprising a plurality of variant nucleic acid templates comprising candidate target sites for a nuclease described herein, wherein each variant nucleic acid template comprises: (i) a first nucleotide sequence encoding a nuclease operably linked to a first promoter; and (ii) a second nucleotide sequence comprising a detection sequence described herein and a candidate target site for the nuclease, wherein upon expression of the nuclease, the nuclease cleaves one or more nucleic acid templates producing a population of cleaved nucleic acid templates, each comprising the detection sequence and a predetermined cleaved end to which an oligonucleotide capture probe described herein specific for the predetermined cleaved end can ligate.

In another aspect, the disclosure provides an emulsion comprising a plurality of droplets comprising a plurality of variant nucleic acid templates comprising candidate target sites for a nuclease described herein, wherein each droplet comprises a unique nucleic acid template comprising: (i) a first nucleotide sequence encoding a nuclease operably linked to a first promoter; and (ii) a second nucleotide sequence comprising a detection sequence described herein and a candidate target site for the nuclease.

In another aspect, the disclosure provides an emulsion comprising a plurality of droplets comprising a plurality of variant nucleic acid templates comprising candidate target sites for a nuclease described herein, wherein each droplet comprises a unique nucleic acid template comprising: (i) a first nucleotide sequence encoding a nuclease operably linked to a first promoter; and (ii) a second nucleotide sequence comprising a detection sequence described herein and a candidate target site for the nuclease, wherein upon expression of the nuclease, the nuclease cleaves one or more nucleic acid templates producing a population of detectable cleaved nucleic acid templates comprising the detection sequence, wherein at least a portion of the detectable cleaved nucleic acid templates comprise a blunt end.

In another aspect, the disclosure provides an emulsion comprising a plurality of droplets comprising a plurality of variant nucleic acid templates comprising candidate target sites for a nuclease described herein, wherein each droplet comprises a unique nucleic acid template comprising: (i) a first nucleotide sequence encoding a nuclease described herein operably linked to a first promoter; and (ii) a second nucleotide sequence comprising a detection sequence described herein and a candidate target site for the nuclease, wherein upon expression of the nuclease, the nuclease cleaves one or more nucleic acid templates producing a population of cleaved nucleic acid templates, each comprising the first detection sequence and a predetermined cleaved end to which an oligonucleotide capture probe described herein specific for the predetermined cleaved end can ligate.

In another aspect, the disclosure provides a composition comprising a plurality of cleaved variant nucleic acid templates and a plurality of uncleaved variant nucleic acid templates, wherein each uncleaved variant nucleic acid template comprises (i) a first nucleotide sequence encoding a nuclease described herein; a second nucleotide sequence comprising a detection sequence described herein and a candidate target site for the nuclease; and wherein at least one cleaved variant nucleic acid template comprises (i) the second nucleotide sequence lacking the candidate target site for the nuclease; and (ii) an oligonucleotide capture probe described herein, wherein the oligonucleotide capture probe is ligated to the second nucleotide sequence by ligation to a predetermined cleaved end of the second nucleotide sequence. In some embodiments, the cleaved and uncleaved variant nucleic acid templates are derived from or produced from a library of nucleic acid templates described herein.

In another aspect, the disclosure provides a system for identifying a nuclease variant having a desired activity, or for identifying a target site for a nuclease described herein, and/or for identifying a guide RNA for an RNA-guided nuclease described herein, comprising an emulsified library described herein and an oligonucleotide capture probe described herein.

In another aspect, the disclosure provides a composition comprising a droplet, the droplet comprising: a variant nucleic acid template comprising (i) a first nucleotide sequence encoding at least one of an RNA guided nuclease and a guide RNA; (ii) a second nucleotide sequence comprising a detection sequence and a candidate target site for the nuclease; and an RNA guided nuclease, if the first nucleotide sequence does not encode an RNA guided nuclease, or a guide RNA if the first nucleotide sequence does not encode a guide RNA.

In another aspect, the disclosure provides methods comprising the steps of: (a) emulsifying an RNA guided nuclease and a library comprising a plurality of variant nucleic acid templates to form droplets, wherein each variant nucleic acid template comprises: (i) a first nucleotide sequence encoding a guide RNA operably linked to a first promoter; and (ii) a second nucleotide sequence comprising a detection sequence and a candidate target site for the nuclease; and wherein each of the plurality of the droplets comprises a unique variant nucleic acid template; (b) subjecting the plurality of the droplets to conditions favorable for nuclease cleavage of the target site to produce a population of cleaved nucleic acid templates, each cleaved nucleic acid template comprising the first nucleotide sequence and a predetermined cleaved end; and (c) ligating the cleaved nucleic acid templates with at least one oligonucleotide capture probe specific for the predetermined cleaved end to produce a plurality of ligation products.

In another aspect, the disclosure provides methods comprising the steps of: (a) emulsifying an RNA guided nuclease and a library comprising a plurality of variant nucleic acid templates to form droplets, wherein each variant nucleic acid template comprises: (i) a first nucleotide sequence encoding a variant of a guide RNA operably linked to a first promoter; and (ii) a second nucleotide sequence comprising a target site for the guide RNA; and wherein each of a plurality of the droplets comprises a unique nucleic acid template; (b) expressing the guide RNA variants in the plurality of the droplets to form nuclease/guide RNA variant complexes; (c) subjecting the plurality of the droplets to conditions favorable for cleavage of the target site by a plurality of nuclease/guide RNA complexes to produce a population of cleaved nucleic acid templates, each cleaved nucleic acid template comprising the first nucleotide sequence and a predetermined cleaved end; (d) ligating the cleaved nucleic acid templates with an oligonucleotide capture probe specific for the predetermined cleaved end to produce a plurality of ligation products; and (e) identifying at least one first nucleotide sequence encoding a guide RNA variant in at least one ligation product.

In another aspect, the disclosure provides methods comprising the steps of: (a) emulsifying an RNA guided nuclease and a library comprising a plurality of variant nucleic acid templates to form droplets, wherein each variant nucleic acid template comprises: (i) a first nucleotide sequence comprising a target site for the nuclease; and (ii) a second nucleotide sequence adjacent the target site, the second nucleotide sequence comprising a variant of a PAM; and wherein each of a plurality of droplets comprises a unique nucleic acid template; (b) subjecting the plurality of the droplets to conditions favorable for nuclease cleavage of the target site to produce a population of cleaved nucleic acid templates, each cleaved nucleic acid template comprising the second nucleotide sequence and a predetermined cleaved end; (c) ligating the cleaved nucleic acid templates with an oligonucleotide capture probe specific for the predetermined cleaved end to produce a plurality of ligation products; and (d) identifying at least one second nucleotide sequence comprising a PAM variant in at least one ligation product.

In another aspect, the disclosure provides methods comprising the steps of: (a) emulsifying an RNA guided nuclease and a library comprising a plurality of variant nucleic acid templates to form droplets, wherein each variant nucleic acid template comprises: (i) a first nucleotide sequence comprising a candidate target site for the nuclease and comprising a unique molecular identifier; and wherein each of a plurality of the droplets comprises a unique nucleic acid template; (b) subjecting the plurality of the droplets to conditions favorable for nuclease cleavage of the candidate target site to produce a population of cleaved nucleic acid templates, each cleaved nucleic acid template comprising a unique molecular identifier and a predetermined cleaved end; (c) ligating the cleaved nucleic acid templates with an oligonucleotide capture probe specific for the predetermined cleaved end to produce a plurality of ligation products; and (d) identifying at least one unique molecular identifier associated with a candidate target site in at least one ligation product.

**BRIEF DESCRIPTION OF THE DRAWING**

**Figure 1** depicts a schematic showing the elements of an exemplary nucleic acid template.

**Figure 2** depicts a schematic showing steps of an exemplary emulsion based screening method.

**Figure 3** depicts a schematic showing ligation of exemplary oligonucleotide capture probes to cleavage products.

**Figure 4** depicts results from WT cas9 (left group) and libraries of mutant cas9 (right grouping) subjected to emulsified in vitro transcription and translation, purified, and ligated using T4 ligase to a variety of oligonucleotide capture probes with either blunt ends ("blunt") or differing overhangs of lengths (shown in Figure 3).

**Figure 5** depicts results from WT cas9 (left group) and libraries of mutant cas9 (right grouping) subjected to emulsified in vitro transcription and translation, purified, and ligated using E. coli ligase to a variety of oligonucleotide capture probes with either blunt ends ("blunt") or differing overhangs of lengths (shown in Figure 3).

## DEFINITIONS

Throughout the specification, several terms are employed that are defined in the following paragraphs. Other definitions may also found within the body of the specification. In this application, unless otherwise clear from context, (i) the term "a" may be understood to mean "at least one"; (ii) the term "or" may be understood to mean "and/or"; (iii) the terms "comprising" and "including" may be understood to encompass itemized components or steps whether presented by themselves or together with one or more additional components or steps; and (iv) the terms "about" and "approximately" may be understood to permit standard variation as would be understood by those of ordinary skill in the art; and (v) where ranges are provided, endpoints are included.

As used herein, the terms "about" and "approximately," in reference to a number, is used herein to include numbers that fall within a range of 20%, 10%, 5%, or 1% in either direction (greater than or less than) of the number unless otherwise stated or otherwise evident from the context (except where such number would exceed 100% of a possible value).

"Cleavage", as used herein, refers to the breakage of the covalent backbone of a DNA molecule. Cleavage can be initiated by a variety of methods including, but not limited to, enzymatic or chemical hydrolysis of a phosphodiester bond. Both single-stranded cleavage and double-stranded cleavage are possible, and double-stranded cleavage can occur as a result of two distinct single-stranded cleavage events. DNA cleavage can result in the production of either blunt ends or cohesive ends.

As used herein, the term "degenerate," when used to refer to an oligonucleotide or nucleotide sequence, refers to one or more positions which may contain any of a plurality of different bases. Degenerate residues within an oligonucleotide or nucleotide sequence are denoted by standard IUPAC nucleic acid notation, as shown below:

| Character | Degenerate Bases |
|:---:|:---:|
| K | G or T/U |
| M | A or C |
| R | A or G |
| Y | C or T/U |
| S | CorG |
| W | A or T/U |
| B | C, G or T/U |
| V | A, C or G |
| H | A, C or T/U |
| D | A, G or T/U |
| N | A, C, G or T/U |

Unless otherwise specified, a degenerate residue does not imply a random or equal distribution of possible bases, e.g., an "N" residue does not denote an equal distribution of A, C, G and/or T/U residues.

As used herein, the term "detecting" a nucleic acid molecule or fragment thereof refers to determining the presence of the nucleic acid molecule, typically when the nucleic acid molecule or fragment thereof has been fully or partially separated from other components of a sample or composition, and also can include determining the charge-to-mass ratio, the mass, the amount, the absorbance, the fluorescence, or other property of the nucleic acid molecule or fragment thereof.

The term "emulsion", as used herein, is in accordance with the meaning normally assigned thereto in the art and further described herein. Generally, an emulsion may be produced from any suitable stable combination of immiscible liquids. Typically, an emulsion of the present disclosure has an aqueous phase that contains molecular components, as the dispersed phase present in the form of finely divided aqueous droplets (the disperse, internal or discontinuous phase), and further comprises a hydrophobic, liquid phase (an "oil") as the matrix in which these droplets are suspended (the continuous or external phase). Such emulsions are termed herein "water-in-oil" (w/o). Advantageously, the entire, or almost the entire, aqueous phase containing the molecular components is compartmentalized in discrete droplets (the internal phase). The hydrophobic oil phase generally contains none of the biochemical components and hence is inert.

As used herein, the term "expression" of a nucleic acid sequence refers to the generation of any gene product from the nucleic acid sequence. In some embodiments, a gene product can be a transcript. In some embodiments, a gene product can be a polypeptide. In some embodiments, expression of a nucleic acid sequence involves one or more of the following: (1) production of an RNA template from a DNA sequence (e.g., by transcription); (2) processing of an RNA transcript (e.g., by splicing, editing, 5' cap formation, and/or 3' end formation); (3) translation of an RNA into a polypeptide or protein; and/or (4) post-translational modification of a polypeptide or protein.

The term "library", as used herein in the context of nucleic acids or proteins, refers to a population of two or more different nucleic acids or proteins, respectively. In some embodiments, a library of nucleic acid templates comprises at least two nucleic acid molecules comprising different sequences encoding nucleases, at least two nucleic acid molecules comprising different sequences encoding guide RNAs, at least two nucleic acid molecules comprising different PAMs, and/or at least two nucleic acid molecules comprising different target sites. In some embodiments, a library comprises at least $10^1$, at least $10^2$, at least $10^3$, at least $10^4$, at least $10^5$, at least $10^6$, at least $10^7$, at least $10^8$, at least $10^9$, at least $10^{10}$, at least $10^{11}$, at least $10^{12}$, at least $10^{13}$, at least $10^{14}$, or at least $10^{15}$ different nucleic acid templates. In some embodiments, the members of the library may comprise randomized sequences, for example, fully or partially randomized sequences. In some embodiments, the library comprises nucleic acid molecules that are unrelated to each other, e.g., nucleic acids comprising fully randomized sequences. In other embodiments, at least some members of the library may be related, for example, they may be variants or derivatives of a particular sequence.

As used herein, the terms "ligation", "ligating", and grammatical equivalents thereof refer to forming a covalent bond or linkage between the termini of two or more nucleic acids, e.g., oligonucleotides and/or polynucleotides, typically in a template-driven reaction. The nature of the bond or linkage may vary widely and the ligation may be carried out enzymatically or chemically. As used herein, ligations are usually carried out enzymatically to form a phosphodiester linkage between a 5' carbon terminal nucleotide of one oligonucleotide with a 3' carbon of another nucleotide. The term "ligation" also encompasses non-enzymatic formation of phosphodiester bonds, as well as the formation of non-phosphodiester covalent bonds between the ends of oligonucleotides, such as phosphorothioate bonds, disulfide bonds, and the like.

As used herein, the term "nuclease" refers to a polypeptide capable of cleaving the phosphodiester bonds between the nucleotide subunits of nucleic acids; the term "endonuclease" refers to a polypeptide capable of cleaving the phosphodiester bond within a polynucleotide chain.

As used herein, the terms "nucleic acid", "nucleic acid molecule" or "polynucleotide" are used herein interchangeably. They refer to a polymer of deoxyribonucleotides or ribonucleotides in either single- or double-stranded form, and unless otherwise stated, encompass known analogs of natural nucleotides that can function in a similar manner as naturally occurring nucleotides. The terms encompass nucleic acid-like structures with synthetic backbones, as well as amplification products. DNAs and RNAs are both polynucleotides. The polymer may include natural nucleosides (i.e., adenosine, thymidine, guanosine, cytidine, uridine, deoxyadenosine, deoxythymidine, deoxyguanosine, and deoxycytidine), nucleoside analogs (e.g., 2-aminoadenosine, 2-thiothymidine, inosine, pyrrolo-pyrimidine, 3-methyl adenosine, C5-propynylcytidine, C5-propynyluridine, C5-bromouridine, C5-fluorouridine, C5-iodouridine, C5-methylcytidine, 7-deazaadenosine, 7-deazaguanosine, 8-oxoadenosine, 8-oxoguanosine, O(6)-methylguanine, and 2-thiocytidine), chemically modified bases, biologically modified bases (e.g., methylated bases), intercalated bases, modified sugars (e.g., 2'-fluororibose, ribose, 2'-deoxyribose, arabinose, and hexose), or modified phosphate groups (e.g., phosphorothioates and 5'-N-phosphoramidite linkages).

As used herein, the term "oligonucleotide" refers to a string of nucleotides or analogues thereof. Oligonucleotides may be obtained by a number of methods including, for example, chemical synthesis, restriction enzyme digestion or PCR. As will be appreciated by one skilled in the art, the length of an oligonucleotide (i.e., the number of nucleotides) can vary widely, often depending on the intended function or use of the oligonucleotide. Generally, oligonucleotides comprise between about 5 and about 300 nucleotides, for example, between about 15 and about 200 nucleotides, between about 15 and about 100 nucleotides, or between about 15 and about 50 nucleotides. Throughout the specification, whenever an oligonucleotide is represented by a sequence of letters (chosen from the four base letters: A, C, G, and T, which denote adenosine, cytidine, guanosine, and thymidine, respectively), the nucleotides are presented in the 5' to 3' order from the left to the right. In certain embodiments, the sequence of an oligonucleotide includes one or more degenerate residues described herein.

"Operably linked", as used herein, refers to a juxtaposition wherein the components described are in a relationship permitting them to function in their intended manner. A control element, e.g., a promoter, "operably linked" to a functional element is associated in such a way that expression and/or activity of the functional element is achieved under conditions compatible with the control element. In some embodiments, "operably linked" control elements are contiguous (e.g., covalently linked) with the coding elements of interest; in some embodiments, control elements act in trans to or otherwise at a from the functional element of interest.

As used herein, the term "polypeptide" generally has its art-recognized meaning of a polymer of amino acids. The term is also used to refer to specific functional classes of polypeptides, such as, for example, nucleases, antibodies, etc.

As used herein, the term "target site," refers to a nucleic acid sequence that defines a portion of a nucleic acid to which a binding molecule will bind, provided sufficient conditions for binding exist. In some embodiments, a target site is a nucleic acid sequence to which a nuclease described herein binds and/or that is cleaved by such nuclease. In some embodiments, a target site is a nucleic acid sequence to which a guide RNA described herein binds. A target site may be single-stranded or double- stranded. In the context of nucleases that dimerize, for example, nucleases comprising a FokI DNA cleavage domain, a target site typically comprises a left-half site (bound by one monomer of the nuclease), a right-half site (bound by the second monomer of the nuclease), and a spacer sequence between the half sites in which the cut is made. In some embodiments, the left-half site and/or the right -half site is between 10-18 nucleotides long. In some embodiments, either or both half- sites are shorter or longer. In some embodiments, the left and right half sites comprise different nucleic acid sequences. In the context of zinc finger nucleases, target sites may, in some embodiments, comprise two half-sites that are each 6-18 bp long flanking a non-specified spacer region that is 4-8 bp long. In the context of TALENs, target sites may, in some embodiments, comprise two half-sites sites that are each 10-23 bp long flanking a non-specified spacer region that is 10-30 bp long. In the context of RNA-guided (e.g., RNA-programmable) nucleases, a target site typically comprises a nucleotide sequence that is complementary to a guide RNA of the RNA-programmable nuclease, and a protospacer adjacent motif (PAM) at the 3' end or 5' end adjacent to the guide RNA-complementary sequence. For the RNA-guided nuclease Cas9, the target site may be, in some embodiments, 16-24 base pairs plus a 3-6 base pair PAM (e.g., NNN, wherein N represents any nucleotide). Exemplary target sites for RNA-guided nucleases, such as Cas9, are known to those of skill in the art and include, without limitation, NNG, NGN, NAG, and NGG, wherein N represents any nucleotide. In addition, Cas9 nucleases from different species (e.g., S. thermophilus instead of S. pyogenes) recognizes a PAM that comprises the sequence NGGNG. Additional PAM sequences are known, including, but not limited to NNAGAAW and NAAR (see, e.g., Esvelt and Wang, Molecular Systems Biology, 9:641 (2013), For example, the target site of an RNA-guided nuclease, such as, e.g., Cas9, may comprise the structure [Nz]-[PAM], where each N is, independently, any nucleotide, and z is an integer between 1 and 50. In some embodiments, z is at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 25, at least 30, at least 35, at least 40, at least 45, or at least 50. In some embodiments, z is 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48,49, or 50. In some embodiments, Z is 20.

As used herein, the term "variant" refers to an entity that shows significant structural identity with a reference entity but differs structurally from the reference entity in the presence or level of one or more chemical moieties as compared with the reference entity. In many embodiments, a variant also differs functionally from its reference entity. In general, whether a particular entity is properly considered to be a "variant" of a reference entity is based on its degree of structural identity with the reference entity. As will be appreciated by those skilled in the art, any biological or chemical reference entity has certain characteristic structural elements. A variant, by definition, is a distinct chemical entity that shares one or more such characteristic structural elements. To give but a few examples, a polypeptide may have a characteristic sequence element comprising a plurality of amino acids having designated positions relative to one another in linear or three-dimensional space and/or contributing to a particular biological function; a nucleic acid may have a characteristic sequence element comprised of a plurality of nucleotide residues having designated positions relative to on another in linear or three-dimensional space. For example, a variant polypeptide may differ from a reference polypeptide as a result of one or more differences in amino acid sequence and/or one or more differences in chemical moieties (e.g., carbohydrates, lipids, etc.) covalently attached to the polypeptide backbone. In some embodiments, a variant polypeptide shows an overall sequence identity with a reference polypeptide (e.g., a nuclease described herein) that is at least 60%, 65%, 70%, 75%, 80%, 85%, 86%, 87%, 88%, 89%, 90%, 91%, 92%, 93%, 94%, 95%, 96%, 97%, or 99%. Alternatively or additionally, in some embodiments, a variant polypeptide does not share at least one characteristic sequence element with a reference polypeptide. In some embodiments, the reference polypeptide has one or more biological activities. In some embodiments, a variant polypeptide shares one or more of the biological activities of the reference polypeptide, e.g., nuclease activity. In some embodiments, a variant polypeptide lacks one or more of the biological activities of the reference polypeptide. In some embodiments, a variant polypeptide shows a reduced level of one or more biological activities (e.g., nuclease activity) as compared with the reference polypeptide. In some embodiments, a polypeptide of interest is considered to be a "variant" of a parent or reference polypeptide if the polypeptide of interest has an amino acid sequence that is identical to that of the parent but for a small number of sequence alterations at particular positions. Typically, fewer than 20%, 15%, 10%, 9%, 8%, 7%, 6%, 5%, 4%, 3%, 2% of the residues in the variant are substituted as compared with the parent. In some embodiments, a variant has 10, 9, 8, 7, 6, 5, 4, 3, 2, or 1 substituted residue as compared with a parent. Often, a variant has a very small number (e.g., fewer than 5, 4, 3, 2, or 1) number of substituted functional residues (i.e., residues that participate in a particular biological activity). Furthermore, a variant typically has not more than 5, 4, 3, 2, or 1 additions or deletions, and often has no additions or deletions, as compared with the parent. Moreover, any additions or deletions are typically fewer than about 25, about 20, about 19, about 18, about 17, about 16, about 15, about 14, about 13, about 10, about 9, about 8, about 7, about 6, and commonly are fewer than about 5, about 4, about 3, or about 2 residues. In some embodiments, the parent or reference polypeptide is one found in nature.

## DETAILED DESCRIPTION OF CERTAIN EMBODIMENTS

The present disclosure provides, among other things, methods of screening nuclease activity using compartmentalized in vitro transcription and translation, such as an emulsified in vitro transcription and translation system. Discovery of novel protein functionalities is often limited by the screen available for a given function. Libraries of RNA-guided nucleases often must often be screened in single-well assays for cleavage activity, which can be laborious and time-consuming. Methods of the present disclosure, involving compartmentalized or emulsion-based screening, represent an alternative to such known methods, and facilitate evaluation of a given nuclease's target site specificity, provide strategies for selection of suitable unique target sites, and the design or selection of highly specific nucleases for targeted cleavage in the context of a complex genome.

The strategies, methods, libraries, and reagents provided herein can be utilized to analyze the sequence preferences and specificity of any site-specific nuclease, for example, Zinc Finger Nucleases (ZFNs), Transcription Activator-Like Effector Nucleases (TALENs), homing endonucleases, organic compound nucleases, and enediyne antibiotics (e.g., dynemicin, neocarzinostatin, calicheamicin, esperamicin, bleomycin). Suitable nucleases in addition to the ones described herein will be apparent to those of skill in the art based on this disclosure.

Further, the methods, reagents, and strategies provided herein allow those of skill in the art to identify, design, and/or select nucleases with enhanced specificity and/or to minimize the off-target effects of any given nuclease (e.g., site-specific nucleases such as ZFNs, and TALENS as well as RNA- programmable nucleases, for example Cas9). Additionally or alternatively, methods described herein allow identification, design, and/or selection of nucleases that cleave target sites to produce particular cleavage ends, e.g., blunt ends and/or cohesive ends. While of particular relevance to DNA and DNA-cleaving nucleases, the inventive concepts, methods, strategies, and reagents provided herein are not limited in this respect, but can be applied to any nucleic acid:nuclease pair.

Screening nucleases

In some aspects, the present disclosure provides methods of assessing nuclease and/or nuclease variants for ability to cleave a particular target site. The methods provided herein can be used for evaluation of target site preferences and specificity of nucleases that create blunt ends and nucleases that create cohesive ends. In some embodiments, methods of the disclosure can also be used to assess the type of cut a nuclease variant produces (e.g., a blunt end or a cohesive end), e.g., by using an oligonucleotide capture probe that binds to a specific type of cut.

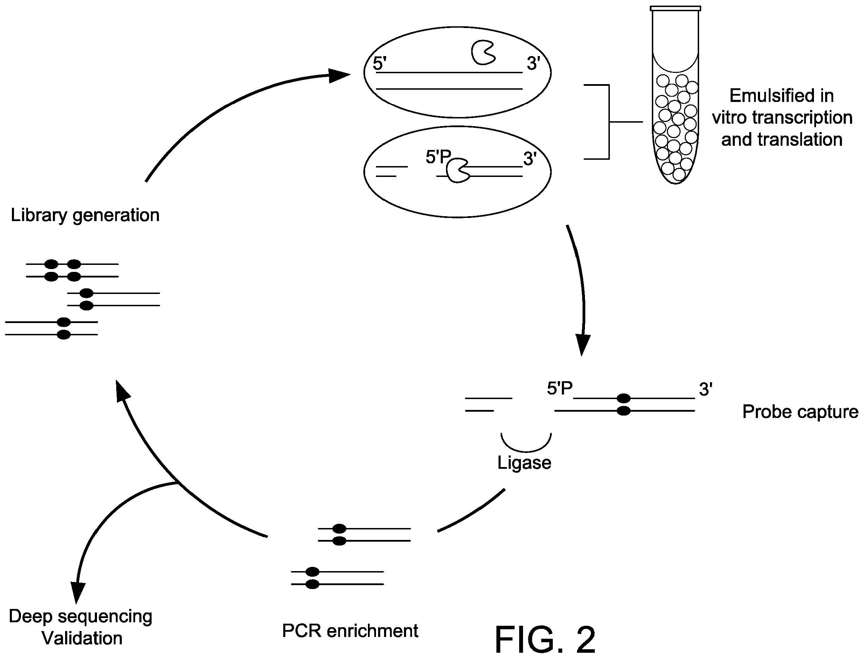

In general, such methods comprise compartmentalizing and/or producing an emulsion comprising a library of nucleic acid templates that comprise both a target site for a given (or reference) nuclease and a nucleotide sequence that encodes a variant of such nuclease. The emulsion is maintained under conditions suitable to express the nuclease variants and for the nuclease variant to bind and cut a target site, and determining which nuclease variant actually cuts a given target site. In general, methods provided herein comprise ligating an oligonucleotide capture probe described herein to nucleic acid templates that have been cut by a nuclease variant, e.g., via 5'-phosphate-dependent ligation. Accordingly, methods provided herein are particularly useful for identifying target sites cut by nucleases that leave a phosphate moiety at the 5'-end of the cut nucleic acid template when cleaving their target site. After ligating an oligonucleotide capture probe to the 5'-end of a cleaved nucleic acid template, the cleaved nucleic acid template (which includes the nucleotide sequence encoding a particular nuclease variant) can be amplified, e.g., by PCR using primers that recognize the oligonucleotide capture probe and the cleaved nucleic acid template. The amplification product can then be sequenced, e.g., to identify the sequence of the nuclease variant that cut the target site. An exemplary method is schematically depicted in Figure 2.

In some embodiments, the method comprises (a) emulsifying a library comprising a plurality of nucleic acid templates to form droplets, wherein each nucleic acid template comprises: (i) a first nucleotide sequence encoding a variant of a nuclease operably linked to a first promoter; and (ii) a second nucleotide sequence comprising a target site for the nuclease; and wherein each of a plurality of the droplets comprises a unique nucleic acid template; (b) expressing the nuclease variants in the plurality of the droplets; (c) subjecting the plurality of the droplets to conditions favorable for nuclease cleavage of the target site to produce a population of cleaved nucleic acid templates, each cleaved nucleic acid template comprising the first nucleotide sequence and a predetermined cleaved end; (d) ligating the cleaved nucleic acid templates with an oligonucleotide capture probe specific for the predetermined cleaved end to produce a plurality of ligation products; and (e) identifying at least one first nucleotide sequence encoding a nuclease variant in at least one ligation product.

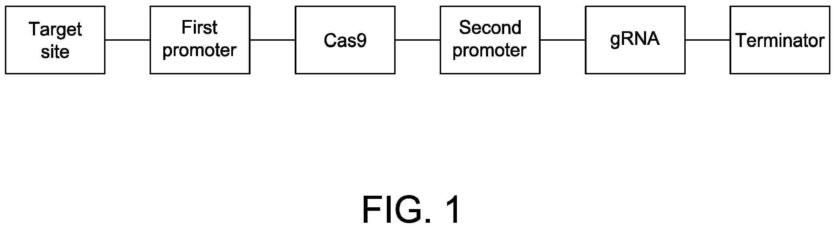

In some embodiments, the library comprises a plurality of nucleic acid templates depicted schematically in Figure 1, which include nucleotide sequences encoding nuclease variants.

In some embodiments, one or more nuclease variants cuts a double-stranded target site and creates blunt ends, and a blunt-ended oligonucleotide capture probe is able to ligate to such blunt ends. In some embodiments, one or more nuclease variants cuts a double-stranded target site and creates an overhang, or cohesive end, for example, a 5'-overhang, and a cohesive-ended oligonucleotide capture probe is able to ligate to such cohesive ends.

In some embodiments, the identifying step (e) comprises amplifying a fragment of the cleaved nucleic acid template (e.g., comprising the first nucleotide sequence encoding a nuclease variant) via PCR using a PCR primer pair that hybridizes with the oligonucleotide capture probe and the cleaved nucleic acid template. In some instances, the PCR parameters can be optimized using known methods to favor amplification of short sequences and disfavor amplification of longer sequences (e.g., by using a short elongation time in the PCR cycle). In some embodiments, size fractionation (e.g., via gel electrophoresis or size exclusion chromatography) can be performed before and/or after amplification.

In some embodiments, the identifying step (e) comprises sequencing the cleaved nucleic acid template, or a copy thereof obtained by amplification, e.g., by PCR. Sequencing methods are well known to those of skill in the art, and any sequencing method can be used.

Suitable conditions for maintaining the emulsion to allow for the nuclease variant to bind and cut a target site will be apparent to those of skill in the art. In some embodiments, suitable conditions do not result in denaturation of the library nucleic acid templates or the nuclease, and allow for the nuclease to exhibit at least 50%, 60%, 70%, 80%, 90%, 95%, 98%, or at least 99% of its nuclease activity.

Some of the methods provided herein allow for the simultaneous assessment of a plurality of nuclease variants for any given target site. Accordingly, data obtained from such methods can be used to compile a list of nuclease variants that cleave a particular target site. In some embodiments, a sequencing method is used to generate quantitative sequencing data, and relative abundance of cleavage of a particular target site by a particular nuclease variant can be determined.

Screening guide RNAs

In some aspects, the present disclosure provides methods of assessing RNA-guided nucleases for ability to cleave a particular target site. In some embodiments, methods provided herein can be used for evaluation of ability of guide RNA variants to direct cutting of a target site by an RNA-guided nuclease. In some embodiments, methods of the disclosure can also be used to assess the type of cut a nuclease produces (e.g., a blunt end or a cohesive end), e.g., by using an oligonucleotide capture probe that binds to a specific type of cut.

In general, such methods comprise compartmentalizing and/or producing an emulsion comprising a library of nucleic acid templates that comprise a target site for a given (or reference) nuclease, a nucleotide sequence that encodes the nuclease, and a nucleotide sequence that encodes a variant of a guide RNA. The emulsion is maintained under conditions suitable for the nuclease to bind and cut a target site, and determining which guide RNA variant mediated cutting of a given target site. In general, methods provided herein comprise ligating an oligonucleotide capture probe described herein to nucleic acid templates that have been cut by the nuclease, e.g., via 5'-phosphate-dependent ligation. Accordingly, methods provided herein are particularly useful for identifying target sites cut by nucleases that leave a phosphate moiety at the 5'-end of the cut nucleic acid template when cleaving their target site. After ligating an oligonucleotide capture probe to the 5'-end of a cleaved nucleic acid template, the cleaved nucleic acid template (which includes the nucleotide sequence encoding a particular guide RNA variant) can be amplified, e.g., by PCR using primers that recognize the oligonucleotide capture probe and the cleaved nucleic acid template. The amplification product can then be sequenced, e.g., to identify the sequence of the guide RNA variant that mediated cutting of the target site. An exemplary method is schematically depicted in Figure 2.

In some embodiments, the method comprises (a) emulsifying a library comprising a plurality of nucleic acid templates to form droplets, wherein each nucleic acid template comprises: (i) a first nucleotide sequence encoding a nuclease operably linked to a first promoter; (ii) a second nucleotide sequence encoding a variant of a guide RNA operably linked to a second promoter; and (iii) a third nucleotide sequence comprising a target site for the guide RNA; and wherein each of a plurality of the droplets comprises a unique nucleic acid template; (b) expressing the nuclease and guide RNA variants in the plurality of the droplets to form nuclease/guide RNA variant complexes; (c) subjecting the plurality of the droplets to conditions favorable for cleavage of the target site by a plurality of nuclease/guide RNA complexes to produce a population of cleaved nucleic acid templates, each cleaved nucleic acid template comprising the second nucleotide sequence and a predetermined cleaved end; (d) ligating the cleaved nucleic acid templates with an oligonucleotide capture probe specific for the predetermined cleaved end to produce a plurality of ligation products; and (e) identifying at least one second nucleotide sequence encoding a guide RNA variant in at least one ligation product.

In some embodiments, the library comprises a plurality of nucleic acid templates depicted schematically in Figure 1, which include nucleotide sequences encoding gRNA variants.

In some embodiments, one or more guide RNA variants mediates cutting of a double-stranded target site and creates blunt ends, and a blunt-ended oligonucleotide capture probe is able to ligate to such blunt ends. In some embodiments, one or more guide RNA variants mediates cutting of a double-stranded target site and creates an overhang, or cohesive end, for example, a 5'-overhang, and a cohesive-ended oligonucleotide capture probe is able to ligate to such cohesive ends.

In some embodiments, the identifying step (e) comprises amplifying a fragment of the cleaved nucleic acid template (e.g., comprising the second nucleotide sequence encoding a guide RNA variant) via PCR using a PCR primer pair that hybridizes with the oligonucleotide capture probe and the cleaved nucleic acid template. In some instances, the PCR parameters can be optimized using known methods to favor amplification of short sequences and disfavor amplification of longer sequences (e.g., by using a short elongation time in the PCR cycle). In some embodiments, size fractionation (e.g., via gel electrophoresis or size exclusion chromatography) can be performed before and/or after amplification.

In some embodiments, the identifying step (e) comprises sequencing the cleaved nucleic acid template, or a copy thereof obtained by amplification, e.g., by PCR. Sequencing methods are well known to those of skill in the art, and any sequencing method can be used.

Suitable conditions for maintaining the emulsion to allow for the nuclease to bind and cut a target site will be apparent to those of skill in the art. In some embodiments, suitable conditions do not result in denaturation of the library nucleic acid templates or the nuclease, and allow for the nuclease to exhibit at least 50%, 60%, 70%, 80%, 90%, 95%, 98%, or at least 99% of its nuclease activity.

Some of the methods provided herein allow for the simultaneous assessment of a plurality of guide RNA variants for any given target site. Accordingly, data obtained from such methods can be used to compile a list of guide RNA variants that mediate cleaving of a particular target site. In some embodiments, a sequencing method is used to generate quantitative sequencing data, and relative abundance of cleavage of a particular target site mediated by a particular guide RNA variant can be determined.

Screening PAMs

In some aspects, the present disclosure provides methods of assessing RNA-guided nucleases for ability to cleave a particular target site. In some embodiments, methods provided herein can be used for evaluation of ability of PAM variants to direct cutting of a target site by an RNA-guided nuclease. In some embodiments, methods of the disclosure can also be used to assess the type of cut a nuclease produces (e.g., a blunt end or a cohesive end), e.g., by using an oligonucleotide capture probe that binds to a specific type of cut.

In general, such methods comprise compartmentalizing and/or producing an emulsion comprising a library of nucleic acid templates that comprise a target site for a given (or reference) nuclease, a nucleotide sequence that encodes the nuclease, and a nucleotide sequence comprising a variant of a PAM, adjacent the target site. The emulsion is maintained under conditions suitable for the nuclease to bind and cut a target site, and determining which PAM variant mediated cutting of a given target site. In general, methods provided herein comprise ligating an oligonucleotide capture probe described herein to nucleic acid templates that have been cut by the nuclease, e.g., via 5'-phosphate-dependent ligation. Accordingly, methods provided herein are particularly useful for identifying target sites cut by nucleases that leave a phosphate moiety at the 5'-end of the cut nucleic acid template when cleaving their target site. After ligating an oligonucleotide capture probe to the 5'-end of a cleaved nucleic acid template, the cleaved nucleic acid template (which includes the nucleotide sequence comprising a particular PAM variant) can be amplified, e.g., by PCR using primers that recognize the oligonucleotide capture probe and the cleaved nucleic acid template. The amplification product can then be sequenced, e.g., to identify the sequence of the PAM variant that mediated cutting of the target site.

In some embodiments, the method comprises (a) emulsifying a library comprising a plurality of nucleic acid templates to form droplets, wherein each nucleic acid template comprises: (i) a first nucleotide sequence encoding a nuclease operably linked to a first promoter; (ii) a second nucleotide sequence comprising a target site for the nuclease; and (iii) a third nucleotide sequence adjacent the target site, the third nucleotide sequence comprising a variant of a PAM; and wherein each of a plurality of the droplets comprises a unique nucleic acid template; (b) expressing the nuclease in the plurality of the droplets; (c) subjecting the plurality of the droplets to conditions favorable for nuclease cleavage of the target site to produce a population of cleaved nucleic acid templates, each cleaved nucleic acid template comprising the third nucleotide sequence and a predetermined cleaved end; (d) ligating the cleaved nucleic acid templates with an oligonucleotide capture probe specific for the predetermined cleaved end to produce a plurality of ligation products; and (e) identifying at least one third nucleotide sequence comprising a PAM variant in at least one ligation product.

In some embodiments, the library comprises a plurality of nucleic acid templates depicted schematically in Figure 1, which further include nucleotide sequences comprising PAM variants adjacent the target site.

In some embodiments, one or more PAM variants mediates cutting of a double-stranded target site and creates blunt ends, and a blunt-ended oligonucleotide capture probe is able to ligate to such blunt ends. In some embodiments, one or more PAM variants mediates cutting of a double-stranded target site and creates an overhang, or cohesive end, for example, a 5'-overhang, and a cohesive-ended oligonucleotide capture probe is able to ligate to such cohesive ends.

In some embodiments, the identifying step (e) comprises amplifying a fragment of the cleaved nucleic acid template (e.g., comprising the third nucleotide sequence comprising a PAM variant) via PCR using a PCR primer pair that hybridizes with the oligonucleotide capture probe and the cleaved nucleic acid template. In some instances, the PCR parameters can be optimized using known methods to favor amplification of short sequences and disfavor amplification of longer sequences (e.g., by using a short elongation time in the PCR cycle). In some embodiments, size fractionation (e.g., via gel electrophoresis or size exclusion chromatography) can be performed before and/or after amplification.

In some embodiments, the identifying step (e) comprises sequencing the cleaved nucleic acid template, or a copy thereof obtained by amplification, e.g., by PCR. Sequencing methods are well known to those of skill in the art, and any sequencing method can be used.

Suitable conditions for maintaining the emulsion to allow for the nuclease to bind and cut a target site will be apparent to those of skill in the art. In some embodiments, suitable conditions do not result in denaturation of the library nucleic acid templates or the nuclease, and allow for the nuclease to exhibit at least 50%, 60%, 70%, 80%, 90%, 95%, 98%, or at least 99% of its nuclease activity.

Some of the methods provided herein allow for the simultaneous assessment of a plurality of PAM variants for any given target site. Accordingly, data obtained from such methods can be used to compile a list of PAM variants that mediate cleaving of a particular target site. In some embodiments, a sequencing method is used to generate quantitative sequencing data, and relative abundance of cleavage of a particular target site mediated by a particular PAM variant can be determined.

Screening target sites

In some aspects, the present disclosure provides methods of assessing a nuclease for ability to cleave different target sites. In some embodiments, methods of the disclosure can also be used to assess the type of cut a nuclease produces (e.g., a blunt end or a cohesive end), e.g., by using an oligonucleotide capture probe that binds to a specific type of cut.

In general, such methods comprise compartmentalizing and/or producing an emulsion comprising a library of nucleic acid templates that comprise both candidate target sites for a given (or reference) nuclease, and a nucleotide sequence that encodes the nuclease. The emulsion is maintained under conditions suitable for the nuclease to bind and cut a target site, and determining which candidate target site was cut by the nuclease. In general, methods provided herein comprise ligating an oligonucleotide capture probe described herein to nucleic acid templates that have been cut by the nuclease, e.g., via 5'-phosphate-dependent ligation. Accordingly, methods provided herein are particularly useful for identifying target sites cut by nucleases that leave a phosphate moiety at the 5'-end of the cut nucleic acid template when cleaving their target site. After ligating an oligonucleotide capture probe to the 5'-end of a cleaved nucleic acid template, the cleaved nucleic acid template (which includes the nucleotide sequence comprising a particular candidate target site) can be amplified, e.g., by PCR using primers that recognize the oligonucleotide capture probe and the cleaved nucleic acid template. The amplification product can then be sequenced, e.g., to identify the sequence of the candidate target site that was cut. An exemplary method is schematically depicted in Figure 2.

In some embodiments, the method comprises (a) emulsifying a library comprising a plurality of nucleic acid templates to form droplets, wherein each nucleic acid template comprises: (i) a first nucleotide sequence encoding a nuclease operably linked to a first promoter; and (ii) a second nucleotide sequence comprising a candidate target site for the nuclease and comprising a unique molecular identifier; and wherein each of a plurality of the droplets comprises a unique nucleic acid template; (b) expressing the nuclease in the plurality of the droplets; (c) subjecting the plurality of the droplets to conditions favorable for nuclease cleavage of the candidate target site to produce a population of cleaved nucleic acid templates, each cleaved nucleic acid template comprising a unique molecular identifier and a predetermined cleaved end; (d) ligating the cleaved nucleic acid templates with an oligonucleotide capture probe specific for the predetermined cleaved end to produce a plurality of ligation products; and (e) identifying at least one unique molecular identifier associated with a candidate target site in at least one ligation product.

In some embodiments, the library comprises a plurality of nucleic acid templates depicted schematically in Figure 1, which include nucleotide sequences comprising target site variants.

In some embodiments, the nuclease cuts a double-stranded candidate target site and creates blunt ends, and a blunt-ended oligonucleotide capture probe is able to ligate to such blunt ends. In some embodiments, the nuclease cuts a double-stranded target site and creates an overhang, or cohesive end, for example, a 5'-overhang, and a cohesive-ended oligonucleotide capture probe is able to ligate to such cohesive ends.

In some embodiments, the identifying step (e) comprises amplifying a fragment of the cleaved nucleic acid template (e.g., comprising the second nucleotide sequence comprising a candidate target site) via PCR using a PCR primer pair that hybridizes with the oligonucleotide capture probe and the cleaved nucleic acid template. In some instances, the PCR parameters can be optimized using known methods to favor amplification of short sequences and disfavor amplification of longer sequences (e.g., by using a short elongation time in the PCR cycle). In some embodiments, size fractionation (e.g., via gel electrophoresis or size exclusion chromatography) can be performed before and/or after amplification.

In some embodiments, the identifying step (e) comprises sequencing the cleaved nucleic acid template, or a copy thereof obtained by amplification, e.g., by PCR. Sequencing methods are well known to those of skill in the art, and any sequencing method can be used.

Suitable conditions for maintaining the emulsion to allow for the nuclease to bind and cut a target site will be apparent to those of skill in the art. In some embodiments, suitable conditions do not result in denaturation of the library nucleic acid templates or the nuclease, and allow for the nuclease to exhibit at least 50%, 60%, 70%, 80%, 90%, 95%, 98%, or at least 99% of its nuclease activity.

Some of the methods provided herein allow for the simultaneous assessment of a plurality of candidate or variant target sites. Accordingly, data obtained from such methods can be used to compile a list of target sites that were cleaved by the nuclease. In some embodiments, a sequencing method is used to generate quantitative sequencing data, and relative abundance of cleavage of a particular target site by the nuclease can be determined.

**Nucleic acid templates**

Methods of the present disclosure involve screening nucleic acid templates. In some embodiments, nucleic acid templates are modular, comprising one or more of the following nucleotide sequence components: a target site (or candidate or variant target site) described herein; a nucleotide sequence encoding a nuclease (or nuclease variant) described herein; a nucleotide sequence encoding a guide RNA (or guide RNA variant) described herein; and a PAM (or PAM variant) described herein. Nucleic acid templates can also include one or more additional elements including, e.g., one or more promoters, a transcription terminator sequence, and/or one or more detection sequences. An exemplary nucleic acid template is depicted in Figure 1. Nucleic acid templates are not limited to any particular order of such components (i.e., 5' to 3' of a nucleic acid), and the disclosure is not limited to particular exemplary nucleic acid templates described herein. Production of nucleic acid templates for use in methods of disclosure are within the skill of those in the art.

In some embodiments, a nucleic acid template comprises (i) a target site for a nuclease, and (ii) a nucleotide sequence encoding a nuclease. In some embodiments, a nucleic acid template comprises, 5' to 3', (i) a target site for a nuclease, and (ii) a nucleotide sequence encoding a nuclease. In some embodiments, the nucleic acid template further comprises a detection sequence situated between the target site and the nucleotide sequence encoding the nuclease. In some embodiments, a nucleic acid template comprises, 5' to 3', (i) a nucleotide sequence encoding a nuclease, and (ii) a target site for a nuclease. In some embodiments, the nucleic acid template further comprises a detection sequence situated between the nucleotide sequence encoding the nuclease and the target site.

In some embodiments, a nucleic acid template comprises (i) a target site for a nuclease, (ii) a nucleotide sequence encoding a nuclease, and (iii) a nucleotide sequence encoding a guide RNA. In some embodiments, a nucleic acid template comprises, 5' to 3', (i) a target site for a nuclease, (ii) a nucleotide sequence encoding a nuclease, and (iii) a nucleotide sequence encoding a guide RNA. In some embodiments, a nucleic acid template comprises, 5' to 3', (i) a target site for a nuclease, (ii) a nucleotide sequence encoding a guide RNA, and (iii) a nucleotide sequence encoding a nuclease. In some embodiments, a nucleic acid template comprises, 5' to 3', (i) a nucleotide sequence encoding a guide RNA, (ii) a target site for a nuclease, and (iii) a nucleotide sequence encoding a nuclease. In some embodiments, a nucleic acid template comprises, 5' to 3', (i) a nucleotide sequence encoding a nuclease, (ii) a target site for a nuclease, and (iii) a nucleotide sequence encoding a guide RNA. In some embodiments, a nucleic acid template comprises, 5' to 3', (i) a nucleotide sequence encoding a nuclease, (ii) a nucleotide sequence encoding a guide RNA, and (iii) a target site for a nuclease. In some embodiments, the nucleic acid template further comprises a detection sequence, e.g., situated between the target site and the nucleotide sequence encoding the guide RNA.

In some embodiments, a nucleic acid template comprises (i) a target site for a nuclease, (ii) a nucleotide sequence encoding a nuclease, and (iii) a nucleotide sequence comprising a PAM, adjacent (e.g., 3' or 5' to) the target site. In some embodiments, a nucleic acid template comprises, 5' to 3', (i) a target site for a nuclease, and (ii) a nucleotide sequence comprising a PAM, adjacent (e.g., 3' or 5' to) the target site, and (iii) a nucleotide sequence encoding a nuclease. In some embodiments, a nucleic acid template comprises, 5' to 3', (i) a nucleotide sequence encoding a nuclease, (ii) a target site for a nuclease, and (iii) a nucleotide sequence comprising a PAM, adjacent (e.g., 3' or 5' to) the target site. In some embodiments, the nucleic acid template further comprises a detection sequence situated either 5' to the target site or 3' to the PAM

In some embodiments, a nucleic acid template further comprises one or more unique molecular identifiers associated with a particular variant (e.g., a variant nuclease, variant guide RNA, variant PAM, and/or variant target site).

In some embodiments, a nucleic acid template comprises one or more spacer sequences, e.g., one or more spacer sequences between, 5' to, or 3' to, one or more of the components described herein. Such spacer sequences may, for example, serve to insulate components, provide sites to which amplification and/or sequencing primers can bind, and/or bring the total size of a nucleic acid template to a desirable size.

Detection sequences

Detection sequences, as used herein, refer to sequence elements that may be present on a nucleic acid template and that facilitate recovery and/or detection of nucleic acids, or nucleic acid fragments, containing them. In some embodiments, one or more detection sequences facilitate or mediate capture by an oligonucleotide array and/or facilitate or mediate sequencing, e.g., sequencing of ligation products described herein.

In some embodiments, detection sequences facilitate amplification and/or sequencing. In some embodiments, detection sequences comprise one or more sequences that can be recognized by amplification and/or sequencing primers.

For example, in some embodiments, detection sequences comprise a sequence adapter for use in a sequencing method. In some embodiments, such sequence adapters comprise an amplification primer binding site and a sequencing primer binding site. In some embodiments, such sequence adapters comprising a primer binding site that serves as both an amplification and sequencing primer binding site. In some embodiments, the amplification primer binding site overlaps with the sequencing primer binding site.

In some embodiments, the amplification primer binding site is used for long-range amplification.

In some embodiments, sequence adapters further comprise a marker sequence that marks one end of the adapter.

In some embodiments, sequence adapters further comprise a barcode sequence.

Detection sequences that can be used in the methods described herein are known in the art. For example, sequencing adapters (e.g., MiSeq adapters) (available from Illumina) can be used as detection sequences.

Unique molecular identifiers

In some embodiments, a nucleic acid template can include a unique molecular identifiers (abbreviated as "UMIs" herein). UMIs refer to sequences that can be used to retrieve information about a nucleic acid template, a variant nucleic acid template, or a portion thereof. For example, in methods of the disclosure involving multiple nucleic acid templates each containing a nucleotide sequence encoding a nuclease variant, a nucleotide sequence encoding a guide RNA variant, a nucleotide sequence comprising a PAM variant, and/or a nucleotide sequence comprising a target site variant, each UMI may be associated with a particular variant.

When a UMI is present on a nucleic acid template containing a detection sequence, it is generally positioned within the nucleic acid template such that, after cleavage by a nuclease, the UMI is present and intact on the fragment containing the detection sequence. For example, in some embodiments, the UMI is positioned between a candidate target site and a detection sequence. In some such embodiments, detection of the detection sequence can be used to identify a particular UMI and, e.g., to identify the nuclease, guide RNA, PAM, and/or target site associated with the particular UMI.

In some embodiments, the UMI is a randomly generated sequence.

The size of the UMI in various embodiments may vary. If a library is used, the size of the library and/or the particular protocols and reagents used to generate the library may influence the size of the UMI. For example, in some embodiments, the UMI is n nucleotides long, where $4^n$ is larger than the number of variants in the library. In some embodiments, n is much larger than it needs to be to cover the number of variants in the library.

In some embodiments, the UMI is between eight and 20 nucleotides in length, for example, between 10 and 16 nucleotides in length, such as 10, 11, 12, 13, 14, 15, and 16 nucleotides in length. The production and use of UMIs in various contexts are known in the art.

## Oligonucleotide capture probes

In some embodiments described herein, oligonucleotide capture probes are used to bind to and/or ligate nucleic acid templates, e.g., cleaved nucleic acid templates. In some embodiments, an oligonucleotide capture probe comprises a detection sequence described herein. In some embodiments, an oligonucleotide capture probe comprises a detection sequence at or near one terminal end. In some embodiments, an oligonucleotide capture probe comprises a detection sequence at or near one terminal end, which is opposite a "capture end" distal to the terminal end comprising a detection sequence. The capture end of the oligonucleotide probe may interact with a nucleic acid fragment intended to be captured. For example, in some embodiments, the oligonucleotide capture probe is ligated to another nucleic acid at or near the capture end.

In some embodiments, oligonucleotide capture probes are double-stranded. In some such embodiments, oligonucleotide capture probes comprise at least one blunt end that serves as the capture end. In some embodiments, the at least one blunt end comprises a 5' phosphate.

In some embodiments, oligonucleotide capture probes are double-stranded and comprise an overhang, e.g., a 5' or a 3' overhang, at the capture end. In some embodiments, the overhang is at least partially complementary to an overhang that results from cleavage of a nucleic acid template with by a nuclease described herein.

In some embodiments, an oligonucleotide capture probe comprises one or more additional sequences, such as one or more random barcodes. In some embodiments, random barcodes are not associated with any particular sequence and may be used, e.g., for quality control purposes. For example, in analyzing ligation products comprising an oligonucleotide capture probe comprising a random barcode, the random barcode can be used to assess amplification bias of a particular ligation product. The over- or under- representation of a given random barcode among amplification products may indicate amplification bias. In some embodiments, data associated with such biased amplification products is excluded.

Suitable sizes for the random barcode may vary depending on the embodiment. By way of non-limiting example, in some embodiments, the random barcode is 2, 3, 4, 5, 6, 7, 8, 9, 10, 11, or 12 nucleotides in length.

In some embodiments, an oligonucleotide capture probe comprises one or more Hamming codes, i.e., an error-correcting barcodes. Hamming codes are sequences that can be used, for example, to identify a particular sample when samples are multiplexed. In some embodiments, there are collectively a defined number of possible Hamming codes, such as, by way of non-limiting example, up to 2, 3, 4, 5, 6, 7, 8, 9, 10, 11, 12, 13, 14, 15, 16, 17, 18, 19, or 20 possible Hamming codes.

In some embodiments, in a method as described herein, a plurality of oligonucleotide capture probes each comprising a random barcode, a Hamming code, or both is ligated to cleaved nucleic acid templates. In some embodiments in which the plurality of oligonucleotide capture probes comprise a random barcode, the distribution of randomized barcodes present in ligation products is analyzed for each variant nucleic acid template.

In some embodiments, oligonucleotide capture probes comprise both a detection sequence and a random barcode. In some embodiments, oligonucleotide capture probes comprise a detection sequence, a Hamming code, and a random barcode.

In some embodiments, an oligonucleotide capture probe is any suitable length to enable ligation to a cleaved nucleic acid template described herein, and optionally identification using a detection sequence described herein. In some embodiments, an oligonucleotide capture probe is about 25, 50, 75, 100, 125, 150, 175, 200, 225, 250, 275, 300, or more, nucleotides in length.

## Libraries of nucleic acid templates

In some embodiments, libraries of variant nucleic acid templates are used in the presently disclosed methods. Generally, variant nucleic acid templates comprise a variant portion and a non-variant portion.

In some embodiments, libraries are "barcoded" in that each variant is associated with a unique molecular identifier (UMI), which can be used to retrieve information about the variant portion of the nucleic acid template. In some embodiments, nucleic acid templates in a library vary in the target site for a nuclease, and each UMI be can associated with a particular variant target site, which may be destroyed by cleavage during analysis of a nuclease's cleavage profile.

In some embodiments, libraries are not barcoded. For example, in some methods described herein, the variant portion of the nucleic acid template would remain intact throughout any method of assessing nuclease cleavage profiles, so no barcode is needed.

In some embodiments, a library includes a plurality of nucleic acid templates described herein. In some embodiments, a library is provided comprising nucleic acid templates that comprise nucleotide sequences encoding nuclease variants, nucleotide sequences encoding guide RNA variants, nucleotide sequences comprising PAM variants, and/or nucleotide sequences comprising target site variants, which nucleotide sequences are fully or partially randomized and/or further comprise one or more partially randomized spacer sequences. In some embodiments, partially randomized sequences differ from a consensus sequence by no more than 10%, no more than 15%, no more than 20%, no more than 25%, nor more than 30%, no more than 40%, or no more than 50% on average, distributed binomially. For example, in some embodiments, partially randomized sequences differ from a consensus sequence by more than 5%, but by no more than 10%; by more than 10%, but by no more than 20%; by more than 20%, but by no more than 25%; by more than 5%, but by no more than 20%, and so on. In some embodiments, for example, using partially randomized target sites in a library is useful to increase the concentration of library members comprising target sites that are closely related to a consensus site, for example, that differ from the consensus sites in only one, only two, only three, only four, or only five residues. In some embodiments, a library comprises nucleic acid templates that comprise fully randomized nucleotide sequences encoding nuclease variants, fully randomized nucleotide sequences encoding guide RNA variants, fully randomized nucleotide sequences comprising PAM variants, and/or fully randomized nucleotide sequences comprising target site variants.

In some embodiments, a library of nucleic acid templates is provided that comprises at least $10^5$, at least $10^6$, at least $10^7$, at least $10^8$, at least $10^9$, at least $10^{10}$, at least $10^{11}$, at least $10^{12}$, at least $10^{13}$, at least $10^{14}$, or at least $10^{15}$ different nucleic acid templates. In some embodiments, the library comprises nucleic acid templates of a molecular weight of at least 5 kDa, at least 6 kDa, at least 7 kDa, at least 8 kDa, at least 9 kDa, at least 10 kDa, at least 12 kDa, or at least 15kDa. In some embodiments, the molecular weight of the nucleic acid templates within the library may be larger than 15 kDa. In some embodiments, the library comprises nucleic acid templates within a specific size range, for example, within a range of 5-7 kDa, 5-10 kDa, 8-12 kDa, 10-15 kDa, or 12-15 kDa, or 5-10 kDa or any possible subrange. Suitable methods for enriching nucleic acid molecules of a desired size or excluding nucleic acid molecules of a desired size are well known to those of skill in the art and the disclosure is not limited in this respect.

In some embodiments, libraries used in presently disclosed methods are synthesized according to any suitable method known in the art (see, e.g., http://blog.allelebiotech.com/tag/degenerate-oligos/). In some embodiments, sequence information is determined using methods described herein, and sequence information is supplied to a commercial vendor for production of a library based on supplied specifications. Commercial vendors are known in the art, e.g., Integrated DNA Technologies (Coralville, Iowa).

Alternatively or additionally, the library may be obtained from a mutagenesis method. In some embodiments, the library is obtained by a random mutagenesis method. In some embodiments, the library is obtained by a comprehensive mutagenesis method, e.g., a method that randomly targets a polynucleotide throughout an entire pre-defined target region for mutagenesis.

In some embodiments, the library is obtained by a targeted mutagenesis method, e.g., by mutagenizing the intended variant portion of the nucleic acid template.

In some embodiments, the library is or is obtained from plasmid library. In some embodiments, plasmids in a library are circular. In some such embodiments, circular plasmids are linearized before use in methods of the present disclosure.

### Emulsions

Compartmentalization of gene libraries is known in the art (described in, e.g., WO99/02671; US Publ. No. 20080004436; Miller et al., Nature Methods 3:561-570 (2006)). Generally, emulsions may be produced from any suitable combination of immiscible liquids. Preferably emulsions comprise an aqueous phase which encompasses (a) components required for in vitro transcription and translation; and (b) a library of nucleic acid templates described herein. In the emulsion, the aqueous phase is present in the form of finely divided droplets (the disperse, internal or discontinuous phase). The emulsion further comprises a hydrophobic, immiscible liquid (an "oil") as the matrix in which droplets are suspended (the nondisperse, continuous or external phase). Such emulsions are termed "water-in-oil" (W/O).

Emulsions may be stabilized by addition of one or more surface-active agents (surfactants). These surfactants are termed emulsifying agents and act at the water/oil interface to prevent (or at least delay) separation of the phases. Many oils and many emulsifiers are known in the art and can be used for the generation of water-in-oil emulsions. Suitable oils include, e.g., light white mineral oil and non-ionic surfactants such as sorbitan monooleate (Span80; ICI) and polyoxyethylenesorbitan monooleate (Tween 80; ICI).

The use of anionic surfactants may also be beneficial. Suitable surfactants include, e.g., sodium cholate and sodium taurocholate. In some embodiments, sodium deoxycholate, e.g., at a concentration of 0.5% w/v, or below, is used. Inclusion of such surfactants can increase the expression of the genetic elements and/or the activity of the gene products.

In some embodiments, emulsions are produced using mechanical energy to force the phases together. Various methods can be employed, including, without limitation, use of mechanical devices, including stirrers (such as magnetic stir-bars, propeller and turbine stirrers, paddle devices and whisks), homogenizers (including rotor-stator homogenizers, high-pressure valve homogenizers and jet homogenizers), colloid mills, and ultrasound and "membrane emulsification" devices.

The size of emulsion microcapsules can be varied by those of skill in the art by tailoring the emulsion conditions used to form the emulsion according to requirements of the selection system. The larger the microcapsule (i.e., aqueous droplet) size, the larger is the volume that will be required to encapsulate a given library, since the ultimately limiting factor will be the size of the microcapsule and thus the number of microcapsules possible per unit volume.

In some embodiments, an emulsion includes an in vitro translation system. In some embodiments, an in vitro translation system includes a cell extract, e.g., from bacteria (Zubay, Annu Rev Genet., 7:267-287, 1973; Lesley et al., J Biol. Chem., 266(4):2632-2638), rabbit reticulocytes (Pelham and Jackson, Eur J Biochem., 67(1):247-256, 1976), or wheat germ. Many suitable systems are commercially available (for example from Promega) including some which will allow coupled transcription/translation (such as the bacterial systems and the reticulocyte and wheat germ TNT extract systems from Promega).

**Nucleases**

Methods of the present disclosure are suitable for assessing the cleavage profiles of a variety of nucleases, including both well-known nucleases and less characterized nucleases. Generally, the nuclease is site-specific in that it is known or expected to cleave only at a specific sequence or set of sequences, referred to herein as the nuclease's "target site".

In methods presently disclosed herein, incubation step(s) with the nuclease are generally carried under out under conditions favorable for the cleavage by the nuclease. That is, even though a given candidate target site or variant target site might not actually be cleaved by the nuclease, the incubation conditions are such that the nuclease would have cleaved at least a significant portion (e.g., at least 10%, at least 20%, at least 30% , at least 40%, at least 50%, at least 60%, at least 70%, at least 80%, at least 90%, or at least 95%) of templates containing its known target site. For known and generally well-characterized nucleases, such conditions are generally known in the art and/or can easily be discovered or optimized. For newly discovered nucleases, such conditions can generally be approximated using information about related nucleases that are better characterized (e.g., homologs and orthologs).

In some embodiments, the nuclease is an endonuclease. In some embodiments, the nuclease is a site-specific endonuclease (e.g., a restriction endonuclease, a meganuclease, a transcription activator-like effector nucleases (TALEN), a zinc finger nuclease, etc.).

In some embodiments, the site specificity of a site-specific nuclease is conferred by an accessory molecule. For example, the CRISPR-associated (Cas) nucleases are guided to specific sites by "guide RNAs" or gRNAs as described herein. In some embodiments, the nuclease is an RNA-guided nuclease. In some embodiments, the nuclease is a CRISPR-associated nuclease.

In some embodiments, the nuclease is a homolog or an ortholog of a previously known nuclease, for example, a newly discovered homolog or ortholog.

RNA-guided nucleases

RNA-guided nucleases according to the present disclosure include, but are not limited to, naturally-occurring Class 2 CRISPR nucleases such as Cas9, and Cpfl, as well as other nucleases derived or obtained therefrom. In functional terms, RNA-guided nucleases are defined as those nucleases that: (a) interact with (e.g., complex with) a gRNA; and (b) together with the gRNA, associate with, and optionally cleave or modify, a target region of a DNA that includes (i) a sequence complementary to the targeting domain of the gRNA and, optionally, (ii) an additional sequence referred to as a "protospacer adjacent motif," or "PAM," which is described in greater detail below. As the following examples will illustrate, RNA-guided nucleases can be defined, in broad terms, by their PAM specificity and cleavage activity, even though variations may exist between individual RNA-guided nucleases that share the same PAM specificity or cleavage activity. Skilled artisans will appreciate that some aspects of the present disclosure relate to systems, methods and compositions that can be implemented using any suitable RNA-guided nuclease having a certain PAM specificity and/or cleavage activity. For this reason, unless otherwise specified, the term RNA-guided nuclease should be understood as a generic term, and not limited to any particular type (e.g., Cas9 vs. Cpfl), species (e.g., S. *pyogenes* vs. *S. aureus*) or variation (e.g., full-length vs. truncated or split; naturally-occurring PAM specificity vs. engineered PAM specificity, etc.) of RNA-guided nuclease.

The PAM sequence takes its name from its sequential relationship to the "protospacer" sequence that is complementary to gRNA targeting domains (or "spacers"). Together with protospacer sequences, PAM sequences define target regions or sequences for specific RNA-guided nuclease / gRNA combinations.

Various RNA-guided nucleases may require different sequential relationships between PAMs and protospacers. In general, Cas9s recognize PAM sequences that are 3' of the protospacer as visualized relative to the top or complementary strand:

5'------------------[protospacer]-------------------------3'

3'-------------------------------[PAM]------------------5'

Cpfl, on the other hand, generally recognizes PAM sequences that are 5' of the protospacer:

5'----------------------------[protospacer] ------------------3'

3'-------------------[PAM]---------------------------------5'

In addition to recognizing specific sequential orientations of PAMs and protospacers, RNA-guided nucleases can also recognize specific PAM sequences. *S. aureus* Cas9, for instance, recognizes a PAM sequence of NNGRRT or NNGRRV, wherein the N residues are immediately 3' of the region recognized by the gRNA targeting domain. *S. pyogenes* Cas9 recognizes NGG PAM sequences. And *F. novicida* Cpfl recognizes a TTN PAM sequence. PAM sequences have been identified for a variety of RNA-guided nucleases, and a strategy for identifying novel PAM sequences has been described by Shmakov et al., 2015, Molecular Cell 60, 385-397, November 5, 2015. It should also be noted that engineered RNA-guided nucleases can have PAM specificities that differ from the PAM specificities of reference molecules (for instance, in the case of an engineered RNA-guided nuclease, the reference molecule may be the naturally occurring variant from which the RNA-guided nuclease is derived, or the naturally occurring variant having the greatest amino acid sequence homology to the engineered RNA-guided nuclease).

In addition to their PAM specificity, RNA-guided nucleases can be characterized by their DNA cleavage activity: naturally-occurring RNA-guided nucleases typically form DSBs in target nucleic acids, but engineered variants have been produced that generate only SSBs (discussed above) Ran & Hsu, et al., Cell 154(6), 1380-1389, September 12, 2013 ("Ran"), ) or that that do not cut at all.

### Cas9

Crystal structures have been determined for *S. pyogenes* Cas9 (Jinek et al., Science 343(6176), 1247997, 2014 ("Jinek 2014"), and for *S. aureus* Cas9 in complex with a unimolecular guide RNA and a target DNA (Nishimasu 2014; Anders et al., Nature. 2014 Sep 25;513(7519):569-73 ("Anders 2014"); and Nishimasu 2015).

A naturally occurring Cas9 protein comprises two lobes: a recognition (REC) lobe and a nuclease (NUC) lobe; each of which comprise particular structural and/or functional domains. The REC lobe comprises an arginine-rich bridge helix (BH) domain, and at least one REC domain (e.g., a REC1 domain and, optionally, a REC2 domain). The REC lobe does not share structural similarity with other known proteins, indicating that it is a unique functional domain. While not wishing to be bound by any theory, mutational analyses suggest specific functional roles for the BH and REC domains: the BH domain appears to play a role in gRNA:DNA recognition, while the REC domain is thought to interact with the repeat:anti-repeat duplex of the gRNA and to mediate the formation of the Cas9/gRNA complex.

The NUC lobe comprises a RuvC domain, an HNH domain, and a PAM-interacting (PI) domain. The RuvC domain shares structural similarity to retroviral integrase superfamily members and cleaves the non-complementary (i.e., bottom) strand of the target nucleic acid. It may be formed from two or more split RuvC motifs (such as RuvC I, RuvCII, and RuvCIII in *S. pyogenes and S. aureus*). The HNH domain, meanwhile, is structurally similar to HNN endonuclease motifs, and cleaves the complementary (i.e., top) strand of the target nucleic acid. The PI domain, as its name suggests, contributes to PAM specificity.

While certain functions of Cas9 are linked to (but not necessarily fully determined by) the specific domains set forth above, these and other functions may be mediated or influenced by other Cas9 domains, or by multiple domains on either lobe. For instance, in *S. pyogenes* Cas9, as described in Nishimasu 2014, the repeat:antirepeat duplex of the gRNA falls into a groove between the REC and NUC lobes, and nucleotides in the duplex interact with amino acids in the BH, PI, and REC domains. Some nucleotides in the first stem loop structure also interact with amino acids in multiple domains (PI, BH and REC1), as do some nucleotides in the second and third stem loops (RuvC and PI domains).

### Cpf1

The crystal structure of *Acidaminococcus sp.* Cpfl in complex with crRNA and a double-stranded (ds) DNA target including a TTTN PAM sequence has been solved by Yamano et al. (Cell. 2016 May 5; 165(4): 949-962 ("Yamano"), incorporated by reference herein). Cpfl, like Cas9, has two lobes: a REC (recognition) lobe, and a NUC (nuclease) lobe. The REC lobe includes REC1 and REC2 domains, which lack similarity to any known protein structures. The NUC lobe, meanwhile, includes three RuvC domains (RuvC-I, -II and -III) and a BH domain. However, in contrast to Cas9, the Cpfl REC lobe lacks an HNH domain, and includes other domains that also lack similarity to known protein structures: a structurally unique PI domain, three Wedge (WED) domains (WED-I, -II and -III), and a nuclease (Nuc) domain.

While Cas9 and Cpfl share similarities in structure and function, it should be appreciated that certain Cpfl activities are mediated by structural domains that are not analogous to any Cas9 domains. For instance, cleavage of the complementary strand of the target DNA appears to be mediated by the Nuc domain, which differs sequentially and spatially from the HNH domain of Cas9. Additionally, the non-targeting portion of Cpfl gRNA (the handle) adopts a pseudoknot structure, rather than a stem loop structure formed by the repeat:antirepeat duplex in Cas9 gRNAs.

Nucleic acids encoding RNA-guided nucleases

Nucleic acids encoding RNA-guided nucleases, e.g., Cas9, Cpfl or functional fragments thereof, are provided herein. Exemplary nucleic acids encoding RNA-guided nucleases have been described previously (see, e.g., Cong et al., Science. 2013 Feb 15;339(6121):819-23 ("Cong 2013"); Wang et al., PLoS One. 2013 Dec 31;8(12):e85650 ("Wang 2013"); Mali 2013; Jinek 2012).

In some cases, a nucleic acid encoding an RNA-guided nuclease can be a synthetic nucleic acid sequence. For example, the synthetic nucleic acid molecule can be chemically modified. In certain embodiments, an mRNA encoding an RNA-guided nuclease will have one or more (e.g., all) of the following properties: it can be capped; polyadenylated; and substituted with 5-methylcytidine and/or pseudouridine.

Synthetic nucleic acid sequences can also be codon optimized, e.g., at least one non-common codon or less-common codon has been replaced by a common codon. For example, the synthetic nucleic acid can direct the synthesis of an optimized messenger mRNA, e.g., optimized for expression in a mammalian expression system, e.g., described herein. Examples of codon optimized Cas9 coding sequences are presented in WO 2016/073990 ("Cotta-Ramusino").

In addition, or alternatively, a nucleic acid encoding an RNA-guided nuclease may comprise a nuclear localization sequence (NLS). Nuclear localization sequences are known in the art.

In some embodiments, a nucleic acid encoding an RNA-guided nuclease is or comprises a single exon encoding the RNA-guided nuclease, e.g., Cas9. In some embodiments, the nucleic acid does not include any introns or other codon-optimization modifications. In some embodiments, the nucleic acid encoding the RNA-guided nuclease is or comprises the following single-exon *S. aureus* Cas9 DNA sequence (or variant thereof):

```
ATGAAGCGCAACTACATCCTGGGCCTGGACATTGGTATTACCAGCGTGGGTTACGGC
ATCATCGACTACGAAACCCGCGACGTGATCGATGCAGGTGTGCGCCTGTTTAAGGA
AGCCAATGTTGAGAATAACGAGGGCCGTCGTAGCAAACGCGGCGCACGTCGTCTGA
AACGCCGCCGCCGTCACCGTATTCAGCGTGTGAAAAAACTGCTGTTTGACTACAACC
TGCTGACCGATCATAGTGAGCTGAGCGGTATCAACCCTTATGAAGCCCGCGTTAAAG
GCCTGAGCCAGAAGCTGAGCGAAGAGGAGTTTAGCGCCGCCCTGCTGCATCTGGCA
AAACGCCGCGGCGTTCACAACGTGAACGAAGTGGAGGAAGATACCGGCAATGAGCT
GAGCACCAAAGAGCAGATCAGCCGCAATAGTAAGGCACTGGAGGAAAAGTACGTG
GCAGAACTGCAACTGGAGCGTCTGAAGAAAGATGGTGAGGTGCGTGGTAGCATCAA
TCGCTTCAAGACAAGCGATTATGTGAAAGAGGCGAAACAGCTGCTGAAAGTGCAGA
AGGCCTATCACCAGCTGGACCAGAGTTTCATTGATACCTATATCGACCTGCTGGAAA
```

CCCGTCGTACCTATTACGAGGGCCCGGGTGAAGGTAGCCCGTTCGGCTGGAAGGAT

ATCAAAGAGTGGTACGAGATGTTAATGGGTCACTGCACCTACTTCCCGGAAGAACT

GCGCAGCGTTAAGTATGCCTACAACGCCGATCTGTACAACGCATTAAACGATTTAAA

CAACTTAGTGATCACCCGCGATGAGAACGAGAAACTGGAATATTACGAAAAATTTC

AGATTATTGAGAACGTTTTTAAGCAGAAGAAAAACCGACATTAAACAGATTGCA

AAAGAAATCCTGGTTAACGAGGAAGATATCAAGGGTTATCGCGTTACCAGCACAGG

CAAGCCGGAGTTCACAAACCTGAAGGTGTACCATGACATCAAGGACATCACCGCCC

GTAAGGAGATTATCGAAAACGCAGAGCTGCTGGACCAGATCGCCAAAATCTTAACC

ATCTATCAGAGTAGCGAGGATATTCAAGAGGAGTTAACCAATCTGAACAGTGAACT

GACACAGGAAGAAATCGAACAGATCAGCAATCTGAAGGGTTATACCGGTACACATA

ACCTGAGCCTGAAGGCCATCAATCTGATCCTGGACGAGTTATGGCACACCAATGAC

AACCAGATTGCCATCTTTAACCGCCTGAAGCTGGTGCCGAAGAAGGTGGATCTGAG

CCAGCAAAAGGAGATTCCTACCACCCTGGTGGACGATTTTATTCTGAGCCCGGTGGT

GAAACGCAGCTTTATCCAGAGCATTAAAGTTATTAACGCAATCATTAAGAAATATGG

CTTACCGAACGACATTATCATTGAACTGGCCCGTGAGAAAAATAGCAAAGATGCCC

AGAAGATGATTAATGAAATGCAAAAGCGTAACCGCCAGACCAATGAGCGCATCGAA

GAAATTATTCGCACCACCGGCAAGGAGAATGCAAAATACCTGATTGAGAAAATTAA

GCTGCACGACATGCAAGAGGGTAAGTGCCTGTATAGTCTGGAAGCCATCCCGCTGG

AGGATTTACTGAACAACCCTTTTAATTATGAAGTGGACCATATCATTCCGCGCAGCG

TGAGTTTTGACAACAGCTTCAACAACAAAGTTTTAGTGAAACAGGAAGAGAATAGC

AAGAAGGGTAATCGCACCCCGTTTCAATACCTGAGCAGCAGCGACAGCAAAATCAG

TTACGAAACCTTTAAAAAACATATCCTGAACCTGGCAAAAGGTAAAGGCCGTATCA

GCAAGACCAAAAAGGAGTATCTGCTGGAAGAACGCGATATTAATCGCTTCAGTGTT

CAGAAAGATTTTATTAATCGCAACCTGGTTGATACCCGCTATGCCACACGCGGTCTG

ATGAACTTATTACGCAGTTATTTCCGTGTTAATAATCTGGACGTTAAAGTTAAGAGC

ATCAATGGCGGCTTTACCAGTTTTCTGCGTCGCAAATGGAAATTTAAAAAGGAACGT

AACAAAGGTTATAAACATCATGCAGAGGACGCCCTGATTATCGCCAACGCCGACTTT

ATTTTTAAGGAATGGAAGAAACTGGATAAAGCAAAGAAGGTGATGGAAAATCAGAT

GTTCGAAGAAAACAGGCCGAGAGCATGCCGGAAATCGAGACCGAGCAGGAGTAC

AAGGAGATCTTCATCACCCCGCACCAGATTAAGCATATCAAGGATTTTAAAGATTAC

AAATACAGCCATCGCGTGGATAAAAAACCGAACCGCGAACTGATTAACGACACCCT

GTACAGCACACGCAAAGACGATAAGGGCAATACCTTAATCGTTAACAACCTGAATG
GCCTGTATGACAAGGATAACGACAAGCTGAAGAAACTGATCAACAAGAGTCCGGAA
AAGTTACTGATGTATCACCATGACCCGCAGACCTATCAGAAACTGAAGCTGATCATG
GAGCAGTACGGCGACGAGAAAAATCCGCTGTATAAATATTACGAAGAAACAGGCAA
CTATCTGACCAAATATAGCAAGAAAGATAACGGTCCGGTTATCAAAAAGATTAAAT
ATTACGGCAATAAGCTGAATGCCCACCTGGATATTACCGATGACTACCCTAACAGCC
GCAACAAAGTTGTTAAACTGAGCCTGAAACCGTACCGCTTTGACGTGTATCTGGATA
ACGGCGTTTATAAGTTTGTTACCGTGAAAAATCTGGATGTGATTAAGAAAGAGAACT
ATTACGAAGTGAATAGTAAATGCTATGAAGAAGCAAAGAAGCTGAAAAAGATCAGT
AACCAGGCAGAATTCATCGCAAGTTTCTACAACAACGATTTAATCAAAATTAATGGC
GAACTGTACCGCGTTATTGGTGTTAACAATGATCTGCTGAATCGTATTGAAGTTAAC
ATGATCGATATCACCTATCGCGAGTATCTGGAGAATATGAATGACAAGCGTCCGCCG
CGCATCATTAAAACCATTGCCAGTAAAACCCAAAGCATTAAAAAGTATAGTACAGA
TATTTTAGGTAATCTGTATGAGGTGAAAAGTAAGAAGCATCCGCAGATTATTAAGAA
AGGCTGA (SEQ ID NO:1)

Guide RNA (gRNA) molecules

The terms "guide RNA" and "gRNA" refer to any nucleic acid that promotes the specific association (or "targeting") of an RNA-guided nuclease such as a Cas9 or a Cpfl to a target sequence such as a genomic or episomal sequence in a cell. gRNAs can be unimolecular (comprising a single RNA molecule, and referred to alternatively as chimeric), or modular (comprising more than one, and typically two, separate RNA molecules, such as a crRNA and a tracrRNA, which are usually associated with one another, for instance by duplexing). gRNAs and their component parts are described throughout the literature, for instance in Briner et al. (Molecular Cell 56(2), 333-339, October 23, 2014 ("Briner"),) and in Cotta-Ramusino.

In bacteria and archea, type II CRISPR systems generally comprise an RNA-guided nuclease protein such as Cas9, a CRISPR RNA (crRNA) that includes a 5' region that is complementary to a foreign sequence, and a trans-activating crRNA (tracrRNA) that includes a 5' region that is complementary to, and forms a duplex with, a 3' region of the crRNA. While not intending to be bound by any theory, it is thought that this duplex facilitates the formation of - and is necessary for the activity of - the Cas9/gRNA complex. As type II CRISPR systems were adapted for use in gene editing, it was discovered that the crRNA and tracrRNA could be joined into a single unimolecular or chimeric guide RNA, in one non-limiting example, by means of a four nucleotide (e.g., GAAA) "tetraloop" or "linker" sequence bridging complementary regions of the crRNA (at its 3' end) and the tracrRNA (at its 5' end). (Mali et al. Science. 2013 Feb 15; 339(6121): 823-826 ("Mali 2013"); Jiang et al. Nat Biotechnol. 2013 Mar; 31(3): 233-239 ("Jiang"); and Jinek et al., 2012 Science Aug. 17; 337(6096): 816-821 ("Jinek 2012"),)

Guide RNAs, whether unimolecular or modular, include a "targeting domain" that is fully or partially complementary to a target domain within a target sequence, such as a DNA sequence in the genome of a cell where editing is desired. Targeting domains are referred to by various names in the literature, including without limitation "guide sequences" (Hsu et al., Nat Biotechnol. 2013 Sep; 31(9): 827-832, ("Hsu"),) "complementarity regions" (Cotta-Ramusino), "spacers" (Briner) and generically as "crRNAs" (Jiang). Irrespective of the names they are given, targeting domains are typically 10-30 nucleotides in length, and in certain embodiments are 16-24 nucleotides in length (for instance, 16, 17, 18, 19, 20, 21, 22, 23 or 24 nucleotides in length), and are at or near the 5' terminus of in the case of a Cas9 gRNA, and at or near the 3' terminus in the case of a Cpfl gRNA.

In addition to the targeting domains, gRNAs typically (but not necessarily, as discussed below) include a plurality of domains that may influence the formation or activity of gRNA/Cas9 complexes. For instance, as mentioned above, the duplexed structure formed by first and secondary complementarity domains of a gRNA (also referred to as a repeat:anti-repeat duplex) interacts with the recognition (REC) lobe of Cas9 and can mediate the formation of Cas9/gRNA complexes. (Nishimasu et al., Cell 156, 935-949, February 27, 2014 ("Nishimasu 2014") and Nishimasu et al., Cell 162, 1113-1126, August 27, 2015 ("Nishimasu 2015"),) It should be noted that the first and/or second complementarity domains may contain one or more poly-A tracts, which can be recognized by RNA polymerases as a termination signal. The sequence of the first and second complementarity domains are, therefore, optionally modified to eliminate these tracts and promote the complete in vitro transcription of gRNAs, for instance through the use of A-G swaps as described in Briner, or A-U swaps. These and other similar modifications to the first and second complementarity domains are within the scope of the present disclosure.

Along with the first and second complementarity domains, Cas9 gRNAs typically include two or more additional duplexed regions that are involved in nuclease activity *in vivo* but not necessarily *in vitro.* (Nishimasu 2015). A first stem-loop one near the 3' portion of the second complementarity domain is referred to variously as the "proximal domain," (Cotta-Ramusino) "stem loop 1" (Nishimasu 2014 and 2015) and the "nexus" (Briner). One or more additional stem loop structures are generally present near the 3' end of the gRNA, with the number varying by species: *S. pyogenes* gRNAs typically include two 3' stem loops (for a total of four stem loop structures including the repeat: anti-repeat duplex), while *S. aureus* and other species have only one (for a total of three stem loop structures). A description of conserved stem loop structures (and gRNA structures more generally) organized by species is provided in Briner.

While the foregoing description has focused on gRNAs for use with Cas9, it should be appreciated that other RNA-guided nucleases have been (or may in the future be) discovered or invented which utilize gRNAs that differ in some ways from those described to this point. For instance, Cpfl ("CRISPR from Prevotella and Franciscella 1") is a recently discovered RNA-guided nuclease that does not require a tracrRNA to function. (Zetsche et al., 2015, Cell 163, 759-771 October 22, 2015 ("Zetsche I"),) A gRNA for use in a Cpfl genome editing system generally includes a targeting domain and a complementarity domain (alternately referred to as a "handle"). It should also be noted that, in gRNAs for use with Cpfl, the targeting domain is usually present at or near the 3' end, rather than the 5' end as described above in connection with Cas9 gRNAs (the handle is at or near the 5' end of a Cpfl gRNA).

Those of skill in the art will appreciate, however, that although structural differences may exist between gRNAs from different prokaryotic species, or between Cpfl and Cas9 gRNAs, the principles by which gRNAs operate are generally consistent. Because of this consistency of operation, gRNAs can be defined, in broad terms, by their targeting domain sequences, and skilled artisans will appreciate that a given targeting domain sequence can be incorporated in any suitable gRNA, including a unimolecular or chimeric gRNA, or a gRNA that includes one or more chemical modifications and/or sequential modifications (substitutions, additional nucleotides, truncations, etc.). Thus, for economy of presentation in this disclosure, gRNAs may be described solely in terms of their targeting domain sequences.

More generally, skilled artisans will appreciate that some aspects of the present disclosure relate to systems, methods and compositions that can be implemented using multiple RNA-guided nucleases. For this reason, unless otherwise specified, the term gRNA should be understood to encompass any suitable gRNA that can be used with any RNA-guided nuclease, and not only those gRNAs that are compatible with a particular species of Cas9 or Cpfl. By way of illustration, the term gRNA can, in certain embodiments, include a gRNA for use with any RNA-guided nuclease occurring in a Class 2 CRISPR system, such as a type II or type V or CRISPR system, or an RNA-guided nuclease derived or adapted therefrom.

## Ligating

Some embodiments of presently disclosed methods comprise a step of ligating nucleic acid sequences, e.g., cleaved ends of a nucleic acid template with an oligonucleotide capture probe or a mixture thereof. In some embodiments, the step of ligating is accomplished using a ligase enzyme that acts on nucleic acids, e.g., a DNA and/or RNA ligase. A variety of such ligases are known in the art, many of which are commercially available.

Examples of ligases that may be used in various embodiments of the presently disclosed methods include, but are not limited to, T4 DNA ligase, T3 DNA ligase, T7 DNA ligase, and E. coli ligase.

The type of ligase chosen may depend on the type of cleaved ends present in the cleavage composition and/or the capture end of oligonucleotide capture probe.

For example, if the cleaved ends in the cleavage composition comprise blunt ends, or comprise cleaved ends that are blunted before ligation (e.g., during an additional step of blunting, as described herein), a ligase suitable for ligating blunt ends may be chosen.

For example, if the cleaved ends in the cleavage composition comprise overhangs ("cohesive ends") that will not be blunted before ligation (e.g., during an additional step of blunting, as described herein), a ligase suitable for ligating sticky ends may be chosen.

Some ligases work well for both blunt ends and ends with overhangs, and any of these ligases may be used in methods of the present disclosure. Furthermore, any combination of two or more ligases may also be used during a ligating step.

## Analysis of ligation products

In some embodiments, a plurality of ligation products described herein is analyzed. In some embodiments, a plurality of ligation products is amplified. In some embodiments in which the plurality of ligation products comprises one or more detection sequences, amplification primers that recognize one or more of the detection sequences can be used.

In some such embodiments, amplification products are analyzed. For example, in some embodiments, methods further comprise a step of determining the levels of ligation products. The levels that are determined can be absolute and/or relative.

In some embodiments, methods further comprise a step of calculating a relative abundance of a ligation product. The analysis may comprise nucleic acid sequencing of the ligation product and/or amplification product thereof. As a non-limiting example, next generation (also known as high throughput) can be performed.

In some embodiments, deep sequencing is performed, meaning that each nucleotide is read several times during the sequencing process, for example at a depth of greater than at least 7, at least 10, at least 15, at least 20, or ever greater, wherein depth (D) is calculated as

$$D = N \times L/G \quad \text{(Equation 1)},$$

wherein $N$ is the number of reads, $L$ is the length of the original genome, and G is length of the polynucleotide being sequenced.

In some embodiments, Sanger sequencing is used to analyze at least some of the ligation products and/or amplification products thereof.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described herein.

The disclosure is further illustrated by the following examples. The examples are provided for illustrative purposes only. They are not to be construed as limiting the scope or content of the disclosure in any way.

**EXAMPLES**

Example 1: Emulsified in vitro transcription, translation, and selection of cas9

A cas9 library was screened for ability of cas9 variants to cleave a target site. Emulsified in vitro transcription and translation allows for efficient selection of large libraries of cas9 by compartmentalizing template library DNA and its translated cas9. Because each cas9 variant is isolated within emulsion, active variants can be identified as those that demonstrate cleavage events on the original template.

*Library construction*

A library was generated based on the following nucleic acid template sequence (capital letters indicate the target site, S. aureus cas9 sequence, and gRNA sequence, respectfully; filler sequences additionally included). A schematic of a nucleic acid template is shown in Figure 1.

Nucleic acid template:

```
ccgcggccgcggcggcacctcGCTAACGGATTCACCACTCCaagaattttacgggctgctagcaattaatacgactcac
tatagggtctagaaataattttgtttaactttaagaaggagatatacatATGAAGCGCAACTACATCCTGGGCCTGG
ACATTGGTATTACCAGCGTGGGTTACGGCATCATCGACTACGAAACCCGCGACGTGA
TCGATGCAGGTGTGCGCCTGTTTAAGGAAGCCAATGTTGAGAATAACGAGGGCCGT
CGTAGCAAACGCGGCGCACGTCGTCTGAAACGCCGCCGCCGTCACCGTATTCAGCG
TGTGAAAAAACTGCTGTTTGACTACAACCTGCTGACCGATCATAGTGAGCTGAGCGG
TATCAACCCTTATGAAGCCCGCGTTAAAGGCCTGAGCCAGAAGCTGAGCGAAGAGG
AGTTTAGCGCCGCCCTGCTGCATCTGGCAAAACGCCGCGGCGTTCACAACGTGAAC
GAAGTGGAGGAAGATACCGGCAATGAGCTGAGCACCAAAGAGCAGATCAGCCGCA
ATAGTAAGGCACTGGAGGAAAAGTACGTGGCAGAACTGCAACTGGAGCGTCTGAAG
```

AAAGATGGTGAGGTGCGTGGTAGCATCAATCGCTTCAAGACAAGCGATTATGTGAA
AGAGGCGAAACAGCTGCTGAAAGTGCAGAAGGCCTATCACCAGCTGGACCAGAGTT
TCATTGATACCTATATCGACCTGCTGGAAACCCGTCGTACCTATTACGAGGGCCCGG
GTGAAGGTAGCCCGTTCGGCTGGAAGGATATCAAAGAGTGGTACGAGATGTTAATG
GGTCACTGCACCTACTTCCCGGAAGAACTGCGCAGCGTTAAGTATGCCTACAACGCC
GATCTGTACAACGCATTAAACGATTTAAACAACTTAGTGATCACCCGCGATGAGAAC
GAGAAACTGGAATATTACGAAAAATTTCAGATTATTGAGAACGTTTTAAGCAGAA
GAAAAAACCGACATTAAACAGATTGCAAAGAAATCCTGGTTAACGAGGAAGATA
TCAAGGGTTATCGCGTTACCAGCACAGGCAAGCCGGAGTTCACAAACCTGAAGGTG
TACCATGACATCAAGGACATCACCGCCCGTAAGGAGATTATCGAAAACGCAGAGCT
GCTGGACCAGATCGCCAAAATCTTAACCATCTATCAGAGTAGCGAGGATATTCAAG
AGGAGTTAACCAATCTGAACAGTGAACTGACACAGGAAGAAATCGAACAGATCAGC
AATCTGAAGGGTTATACCGGTACACATAACCTGAGCCTGAAGGCCATCAATCTGATC
CTGGACGAGTTATGGCACACCAATGACAACCAGATTGCCATCTTTAACCGCCTGAAG
CTGGTGCCGAAGAAGGTGGATCTGAGCCAGCAAAAGGAGATTCCTACCACCCTGGT
GGACGATTTTATTCTGAGCCCGGTGGTGAAACGCAGCTTTATCCAGAGCATTAAAGT
TATTAACGCAATCATTAAGAAATATGGCTTACCGAACGACATTATCATTGAACTGGC
CCGTGAGAAAAATAGCAAAGATGCCCAGAAGATGATTAATGAAATGCAAAAGCGTA
ACCGCCAGACCAATGAGCGCATCGAAGAAATTATTCGCACCACCGGCAAGGAGAAT
GCAAAATACCTGATTGAGAAAATTAAGCTGCACGACATGCAAGAGGGTAAGTGCCT
GTATAGTCTGGAAGCCATCCCGCTGGAGGATTTACTGAACAACCCTTTTAATTATGA
AGTGGACCATATCATTCCGCGCAGCGTGAGTTTTGACAACAGCTTCAACAACAAAGT
TTTAGTGAAACAGGAAGAGAATAGCAAGAAGGGTAATCGCACCCCGTTTCAATACC
TGAGCAGCAGCGACAGCAAAATCAGTTACGAAACCTTTAAAAAACATATCCTGAAC
CTGGCAAAAGGTAAAGGCCGTATCAGCAAGACCAAAAAGGAGTATCTGCTGGAAGA
ACGCGATATTAATCGCTTCAGTGTTCAGAAAGATTTTATTAATCGCAACCTGGTTGA
TACCCGCTATGCCACACGCGGTCTGATGAACTTATTACGCAGTTATTTCCGTGTTAAT
AATCTGGACGTTAAAGTTAAGAGCATCAATGGCGGCTTTACCAGTTTTCTGCGTCGC
AAATGGAAATTTAAAAAGGAACGTAACAAAGGTTATAAACATCATGCAGAGGACGC
CCTGATTATCGCCAACGCCGACTTTATTTTTAAGGAATGGAAGAAACTGGATAAAGC
AAAGAAGGTGATGGAAAATCAGATGTTCGAAGAAAAACAGGCCGAGAGCATGCCG
GAAATCGAGACCGAGCAGGAGTACAAGGAGATCTTCATCACCCCGCACCAGATTAA
GCATATCAAGGATTTTAAAGATTACAAATACAGCCATCGCGTGGATAAAAAACCGA
ACCGCGAACTGATTAACGACACCCTGTACAGCACACGCAAAGACGATAAGGGCAAT
ACCTTAATCGTTAACAACCTGAATGGCCTGTATGACAAGGATAACGACAAGCTGAA
GAAACTGATCAACAAGAGTCCGGAAAAGTTACTGATGTATCACCATGACCCGCAGA
CCTATCAGAAACTGAAGCTGATCATGGAGCAGTACGGCGACGAGAAAAATCCGCTG
TATAAATATTACGAAGAAACAGGCAACTATCTGACCAAATATAGCAAGAAAGATAA
CGGTCCGGTTATCAAAAAGATTAAATATTACGGCAATAAGCTGAATGCCCACCTGG
ATATTACCGATGACTACCCTAACAGCCGCAACAAAGTTGTTAAACTGAGCCTGAAAC
CGTACCGCTTTGACGTGTATCTGGATAACGGCGTTTATAAGTTTGTTACCGTGAAAA
ATCTGGATGTGATTAAGAAAGAGAACTATTACGAAGTGAATAGTAAATGCTATGAA
GAAGCAAAGAAGCTGAAAAAGATCAGTAACCAGGCAGAATTCATCGCAAGTTTCTA
CAACAACGATTTAATCAAAATTAATGGCGAACTGTACCGCGTTATTGGTGTTAACAA
TGATCTGCTGAATCGTATTGAAGTTAACATGATCGATATCACCTATCGCGAGTATCT
GGAGAATATGAATGACAAGCGTCCGCCGCGCATCATTAAAACCATTGCCAGTAAAA

CCCAAAGCATTAAAAAGTATAGTACAGATATTTTAGGTAATCTGTATGAGGTGAAA
AGTAAGAAGCATCCGCAGATTATTAAGAAAGGCTGAatgcatccggtaatacgactcactatagggaat
acaagctacttgttcttttttgcaGCTAACGGATTCACCACTCCGTTTTAGTACTCTGGAAACAGAAT
CTACTAAAACAAGGCAAAATGCCGTGTTTATCTCGTCAACTTGTTGGCGAGATTTTT
Tccgctgagcaataactagcataacccccttggggcctctaaacgggtcttgaggggtttttttgacaaagaaagccgggcaatgcccggcttt
ttctcgagatggaacataataacatgtggatggcc (SEQ ID NO:2)

The nucleic acid template included the following components:

Target site: GCTAACGGATTCACCACTCC (SEQ ID NO:3)

PAM: AAGAAT (SEQ ID NO:4)

First T7 promoter: taatacgactcactatagggtctagaaataatttgtttaactttaagaaggagatatacat (SEQ ID NO:5)

SAcas9 ORF :TGAAGCGCAACTACATCCTGGGCCTGGACATTGGTATTACCAG
CGTGGGTTACGGCATCATCGACTACGAAACCCGCGACGTGATCGATGCAGGTGTGC
GCCTGTTTAAGGAAGCCAATGTTGAGAATAACGAGGGCCGTCGTAGCAAACGCGGC
GCACGTCGTCTGAAACGCCGCCGCCGTCACCGTATTCAGCGTGTGAAAAAACTGCTG
TTTGACTACAACCTGCTGACCGATCATAGTGAGCTGAGCGGTATCAACCCTTATGAA
GCCCGCGTTAAAGGCCTGAGCCAGAAGCTGAGCGAAGAGGAGTTTAGCGCCGCCCT
GCTGCATCTGGCAAAACGCCGCGGCGTTCACAACGTGAACGAAGTGGAGGAAGATA
CCGGCAATGAGCTGAGCACCAAAGAGCAGATCAGCCGCAATAGTAAGGCACTGGA
GGAAAAGTACGTGGCAGAACTGCAACTGGAGCGTCTGAAGAAAGATGGTGAGGTGC
GTGGTAGCATCAATCGCTTCAAGACAAGCGATTATGTGAAAGAGGCGAAACAGCTG
CTGAAAGTGCAGAAGGCCTATCACCAGCTGGACCAGAGTTTCATTGATACCTATATC
GACCTGCTGGAAACCCGTCGTACCTATTACGAGGGCCCGGGTGAAGGTAGCCCGTT
CGGCTGGAAGGATATCAAAGAGTGGTACGAGATGTTAATGGGTCACTGCACCTACT
TCCCGGAAGAACTGCGCAGCGTTAAGTATGCCTACAACGCCGATCTGTACAACGCA
TTAAACGATTTAAACAACTTAGTGATCACCCGCGATGAGAACGAGAAACTGGAATA
TTACGAAAAATTTCAGATTATTGAGAACGTTTTTAAGCAGAAGAAAAAACCGACATT
AAAACAGATTGCAAAAGAAATCCTGGTTAACGAGGAAGATATCAAGGGTTATCGCG
TTACCAGCACAGGCAAGCCGGAGTTCACAAACCTGAAGGTGTACCATGACATCAAG
GACATCACCGCCCGTAAGGAGATTATCGAAACGCAGAGCTGCTGGACCAGATCGC
CAAAATCTTAACCATCTATCAGAGTAGCGAGGATATTCAAGAGGAGTTAACCAATCT
GAACAGTGAACTGACACAGGAAGAAATCGAACAGATCAGCAATCTGAAGGGTTATA
CCGGTACACATAACCTGAGCCTGAAGGCCATCAATCTGATCCTGGACGAGTTATGGC
ACACCAATGACAACCAGATTGCCATCTTTAACCGCCTGAAGCTGGTGCCGAAGAAG
GTGGATCTGAGCCAGCAAAAGGAGATTCCTACCACCCTGGTGGACGATTTATTCTG
AGCCCGGTGGTGAAACGCAGCTTTATCCAGAGCATTAAAGTTATTAACGCAATCATT
AAGAAATATGGCTTACCGAACGACATTATCATTGAACTGGCCCGTGAGAAAAATAG
CAAAGATGCCCAGAAGATGATTAATGAAATGCAAAAGCGTAACCGCCAGACCAATG
AGCGCATCGAAGAAATTATTCGCACCACCGGCAAGGAGAATGCAAAATACCTGATT
GAGAAAATTAAGCTGCACGACATGCAAGAGGGTAAGTGCCTGTATAGTCTGGAAGC
CATCCCGCTGGAGGATTTACTGAACAACCCTTTTAATTATGAAGTGGACCATATCAT

TCCGCGCAGCGTGAGTTTTGACAACAGCTTCAACAACAAAGTTTTAGTGAAACAGG
AAGAGAATAGCAAGAAGGGTAATCGCACCCCGTTTCAATACCTGAGCAGCAGCGAC
AGCAAAATCAGTTACGAAACCTTTAAAAAACATATCCTGAACCTGGCAAAAGGTAA
AGGCCGTATCAGCAAGACCAAAAAGGAGTATCTGCTGGAAGAACGCGATATTAATC
GCTTCAGTGTTCAGAAAGATTTTATTAATCGCAACCTGGTTGATACCCGCTATGCCA
CACGCGGTCTGATGAACTTATTACGCAGTTATTCCGTGTTAATAATCTGGACGTTA
AAGTTAAGAGCATCAATGGCGGCTTTACCAGTTTTCTGCGTCGCAAATGGAAATTTA
AAAAGGAACGTAACAAAGGTTATAAACATCATGCAGAGGACGCCCTGATTATCGCC
AACGCCGACTTTATTTTTAAGGAATGGAAGAAACTGGATAAAGCAAAGAAGGTGAT
GGAAAATCAGATGTTCGAAGAAAAACAGGCCGAGAGCATGCCGGAAATCGAGACC
GAGCAGGAGTACAAGGAGATCTTCATCACCCCGCACCAGATTAAGCATATCAAGGA
TTTTAAAGATTACAAATACAGCCATCGCGTGGATAAAAAACCGAACCGCGAACTGA
TTAACGACACCCTGTACAGCACACGCAAAGACGATAAGGGCAATACCTTAATCGTT
AACAACCTGAATGGCCTGTATGACAAGGATAACGACAAGCTGAAGAAACTGATCAA
CAAGAGTCCGGAAAAGTTACTGATGTATCACCATGACCCGCAGACCTATCAGAAAC
TGAAGCTGATCATGGAGCAGTACGGCGACGAGAAAAATCCGCTGTATAAATATTAC
GAAGAAACAGGCAACTATCTGACCAAATATAGCAAGAAAGATAACGGTCCGGTTAT
CAAAAAGATTAAATATTACGGCAATAAGCTGAATGCCCACCTGGATATTACCGATG
ACTACCCTAACAGCCGCAACAAAGTTGTTAAACTGAGCCTGAAACCGTACCGCTTTG
ACGTGTATCTGGATAACGGCGTTTATAAGTTTGTTACCGTGAAAAATCTGGATGTGA
TTAAGAAAGAGAACTATTACGAAGTGAATAGTAAATGCTATGAAGAAGCAAAGAAG
CTGAAAAAGATCAGTAACCAGGCAGAATTCATCGCAAGTTTCTACAACAACGATTT
AATCAAAATTAATGGCGAACTGTACCGCGTTATTGGTGTTAACAATGATCTGCTGAA
TCGTATTGAAGTTAACATGATCGATATCACCTATCGCGAGTATCTGGAGAATATGAA
TGACAAGCGTCCGCCGCGCATCATTAAAACCATTGCCAGTAAAACCCAAAGCATTA
AAAAGTATAGTACAGATATTTTAGGTAATCTGTATGAGGTGAAAAGTAAGAAGCAT
CCGCAGATTATTAAGAAAGGCTGA (SEQ ID NO:6)

<u>second T7 promoter</u>:  taatacgactcactatagggaatacaagctacttgttcttttgca (SEQ ID NO:7)

<u>gRNA target sequence (protospacer)</u>:  GCTAACGGATTCACCACTCC (SEQ ID NO:8)

<u>Tracr</u>:  GTTTTAGTACTCTGGAAACAGAATCTACTAAAACAAGGCAAAATGCCG
TGTTTATCTCGTCAACTTGTTGGCGAGATTTTTT (SEQ ID NO:9)

<u>T7 terminator</u>:  ccgctgagcaataactagcataaccccttggggcctctaaacgggtcttgagggggttttttgacaaagaa
agccgggcaatgcccggctttt (SEQ ID NO:10)

*Probe construction*

The following synthetic double stranded oligonucleotide capture probe was constructed to screen for 3' overhangs on cleavage products. The capital bases are present in the forward strand but are absent in the reverse complemented strand of the probe:

5'-atccgaccctcgcgacttctagagaagaagagtactgacttgagcgctcccagcacttcagccaagttaccaatttcttgtttcc
gaatgacacgCACCAC-3' (SEQ ID NO:11)

*Screening method*

A 50 ml solution was created containing 2.25 ml Span 80, 250 $\mu$l Tween 80, and 47.5 ml mineral oil. 950 $\mu$l of the solution was transferred to a flat-bottomed cryotube with a 3 mm x 8 mm stir bar and placed in a cooled aluminum block on ice. 1.66 fmol of the plasmid library was mixed on ice with NEB PURExpress reagents (10 $\mu$l Solution A, 7.5 $\mu$l Solution B , 0.5 $\mu$l murine RNAse inhibitor, and nuclease free water to 25 $\mu$l). To produce an emulsion, this mixture was gradually added to the oil surfactant mixture in three aliquots of < 10 $\mu$l over 2 minutes while spinning at 1150 rpm in a cold aluminum block. The emulsion mixture was allowed to come to 37°C in a static incubator and incubated for 1-4 hours to allow for in vitro transcription and translation of cas9 variants as well as to allow for cleavage of templates by cas9 variants.

The emulsion was then transferred to a 1.5 ml tube and centrifuged at 13,000 g for 5 minutes at room temperature. The upper phase was discarded. 1 ml of saturated diethyl ether was added to the tube and the tube was vortexed. The upper phase was again discarded, and the step was repeated. The tube was next vacuum centrifuged for 5 minutes followed by DNA purification using the DNA Clean & Concentrator™-5 product (Zymo Research) according to manufacturer's protocol.

Cleaved templates were selected and enriched by the ligation of the oligonucleotide capture probe described above to the exposed phosphorylated cleavage site using T4 DNA Ligase (NEB), T7 Ligase (NEB), or E Coli Ligase (NEB) according to the manufacturer's instructions. Primer sites on the probe and the template DNA were used to PCR amplify the successfully ligated DNA using Phusion Polymerase (NEB). An exemplary expected product of probe ligation and amplification has the following sequence:

```
atccgaccctcgcgacttctagagaagaagagtactgacttgagcgctcccagcacttcagccaagttaccaatttcttgtttccgaatgacac
gCACCACTCCAAGAATtttacgggctgctagcaattaatacgactcactatagggtctagaaataatttgtttaactttaagaag
gagatatacatATGAAGCGCAACTACATCCTGGGCCTGGACATTGGTATTACCAGCGTGGG
TTACGGCATCATCGACTACGAAACCCGCGACGTGATCGATGCAGGTGTGCGCCTGTT
TAAGGAAGCCAATGTTGAGAATAACGAGGGCCGTCGTAGCAAACGCGGCGCACGTC
```

GTCTGAAACGCCGCCGCCGTCACCGTATTCAGCGTGTGAAAAAACTGCTGTTTGACT
ACAACCTGCTGACCGATCATAGTGAGCTGAGCGGTATCAACCCTTATGAAGCCCGC
GTTAAAGGCCTGAGCCAGAAGCTGAGCGAAGAGGAGTTTAGCGCCGCCCTGCTGCA
TCTGGCAAAACGCCGCGGCGTTCACAACGTGAACGAAGTGGAGGAAGATACCGGCA
ATGAGCTGAGCACCAAAGAGCAGATCAGCCGCAATAGTAAGGCACTGGAGGAAAA
GTACGTGGCAGAACTGCAACTGGAGCGTCTGAAGAAAGATGGTGAGGTGCGTGGTA
GCATCAATCGCTTCAAGACAAGCGATTATGTGAAAGAGGCGAAACAGCTGCTGAAA
GTGCAGAAGGCCTATCACCAGCTGGACCAGAGTTTCATTGATACCTATATCGACCTG
CTGGAAACCCGTCGTACCTATTACGAGGGCCCGGGTGAAGGTAGCCCGTTCGGCTG
GAAGGATATCAAAGAGTGGTACGAGATGTTAATGGGTCACTGCACCTACTTCCCGG
AAGAACTGCGCAGCGTTAAGTATGCCTACAACGCCGATCTGTACAACGCATTAAAC
GATTTAAACAACTTAGTGATCACCCGCGATGAGAACGAGAAACTGGAATATTACGA
AAAATTTCAGATTATTGAGAACGTTTTTAAGCAGAAGAAAAAACCGACATTAAAAC
AGATTGCAAAAGAAATCCTGGTTAACGAGGAAGATATCAAGGGTTATCGCGTTACC
AGCACAGGCAAGCCGGAGTTCACAAACCTGAAGGTGTACCATGACATCAAGGACAT
CACCGCCCGTAAGGAGATTATCGAAACGCAGAGCTGCTGGACCAGATCGCCAAAA
TCTTAACCATCTATCAGAGTAGCGAGGATATTCAAGAGGAGTTAACCAATCTGAACA
GTGAACTGACACAGGAAGAAATCGAACAGATCAGCAATCTGAAGGGTTATACCGGT
ACACATAACCTGAGCCTGAAGGCCATCAATCTGATCCTGGACGAGTTATGGCACAC
CAATGACAACCAGATTGCCATCTTTAACCGCCTGAAGCTGGTGCCGAAGAAGGTGG
ATCTGAGCCAGCAAAAGGAGATTCCTACCACCCTGGTGGACGATTTTATTCTGAGCC
CGGTGGTGAAACGCAGCTTTATCCAGAGCATTAAAGTTATTAACGCAATCATTAAGA
AATATGGCTTACCGAACGACATTATCATTGAACTGGCCCGTGAGAAAAATAGCAAA
GATGCCCAGAAGATGATTAATGAAATGCAAAAGCGTAACCGCCAGACCAATGAGCG
CATCGAAGAAATTATTCGCACCACCGGCAAGGAGAATGCAAAATACCTGATTGAGA
AAATTAAGCTGCACGACATGCAAGAGGGTAAGTGCCTGTATAGTCTGGAAGCCATC
CCGCTGGAGGATTTACTGAACAACCCTTTTAATTATGAAGTGGACCATATCATTCCG
CGCAGCGTGAGTTTTGACAACAGCTTCAACAACAAAGTTTTAGTGAAACAGGAAGA
GAATAGCAAGAAGGGTAATCGCACCCCGTTCAATACCTGAGCAGCAGCGACAGCA
AAATCAGTTACGAAACCTTTAAAAAACATATCCTGAACCTGGCAAAAGGTAAAGGC
CGTATCAGCAAGACCAAAAAGGAGTATCTGCTGGAAGAACGCGATATTAATCGCTT
CAGTGTTCAGAAAGATTTTATTAATCGCAACCTGGTTGATACCCGCTATGCCACACG
CGGTCTGATGAACTTATTACGCAGTTATTTCCGTGTTAATAATCTGGACGTTAAAGTT
AAGAGCATCAATGGCGGCTTTACCAGTTTTCTGCGTCGCAAATGGAAATTTAAAAAG
GAACGTAACAAAGGTTATAAACATCATGCAGAGGACGCCCTGATTATCGCCAACGC
CGACTTTATTTTTAAGGAATGGAAGAAACTGGATAAAGCAAAGAAGGTGATGGAAA
ATCAGATGTTCGAAGAAAACAGGCCGAGAGCATGCCGGAAATCGAGACCGAGCA
GGAGTACAAGGAGATCTTCATCACCCGCACCAGATTAAGCATATCAAGGATTTTAA
AGATTACAAATACAGCCATCGCGTGGATAAAAAACCGAACCGCGAACTGATTAACG
ACACCCTGTACAGCACACGCAAAGACGATAAGGGCAATACCTTAATCGTTAACAAC
CTGAATGGCCTGTATGACAAGGATAACGACAAGCTGAAGAAACTGATCAACAAGAG
TCCGGAAAAGTTACTGATGTATCACCATGACCCGCAGACCTATCAGAAACTGAAGCT
GATCATGGAGCAGTACGGCGACGAGAAAATCGCTGTATAAATATTACGAAGAAA
CAGGCAACTATCTGACCAAATATAGCAAGAAGATAACGGTCCGGTTATCAAAAAG
ATTAAATATTACGGCAATAAGCTGAATGCCCACCTGGATATTACCGATGACTACCCT
AACAGCCGCAACAAAGTTGTTAAACTGAGCCTGAAACCGTACCGCTTTGACGTGTAT

CTGGATAACGGCGTTTATAAGTTTGTTACCGTGAAAAATCTGGATGTGATTAAGAAA
GAGAACTATTACGAAGTGAATAGTAAATGCTATGAAGAAGCAAAGAAGCTGAAAA
AGATCAGTAACCAGGCAGAATTCATCGCAAGTTTCTACAACAACGATTTAATCAAA
ATTAATGGCGAACTGTACCGCGTTATTGGTGTTAACAATGATCTGCTGAATCGTATT
GAAGTAACATGATCGATATCACCTATCGCGAGTATCTGGAGAATATGAATGACAA
GCGTCCGCCGCGCATCATTAAAACCATTGCCAGTAAAACCCAAAGCATTAAAAAGT
ATAGTACAGATATTTAGGTAATCTGTATGAGGTGAAAAGTAAGAAGCATCCGCAG
ATTATTAAGAAAGGCTGAATGCATccggtaatacgactcactatagggaatacaagctacttgttctttttgcaGCT
AACGGATTCACCACTCCGTTTTAGTACTCTGGAAACAGAATCTACTAAAACAAGGCA
AAATGCCGTGTTTATCTCGTCAACTTGTTGGCGAGATTTTTTccgctgagcaataactagcataacc
ccttggggcctctaaacgggtcttgaggggttttttgacaaagaaagccgggcaatgcccggctttttCTCGAGATGGAACAT
AATAACATGTGGATGGCC (SEQ ID NO: 12)

Example 2: Assessing ligation of capture probes to cleavage products by ligases

In the present Example, ability of T4 ligase or *E. coli* ligase to ligate various oligonucleotide capture probe to various cleavage products was assessed.

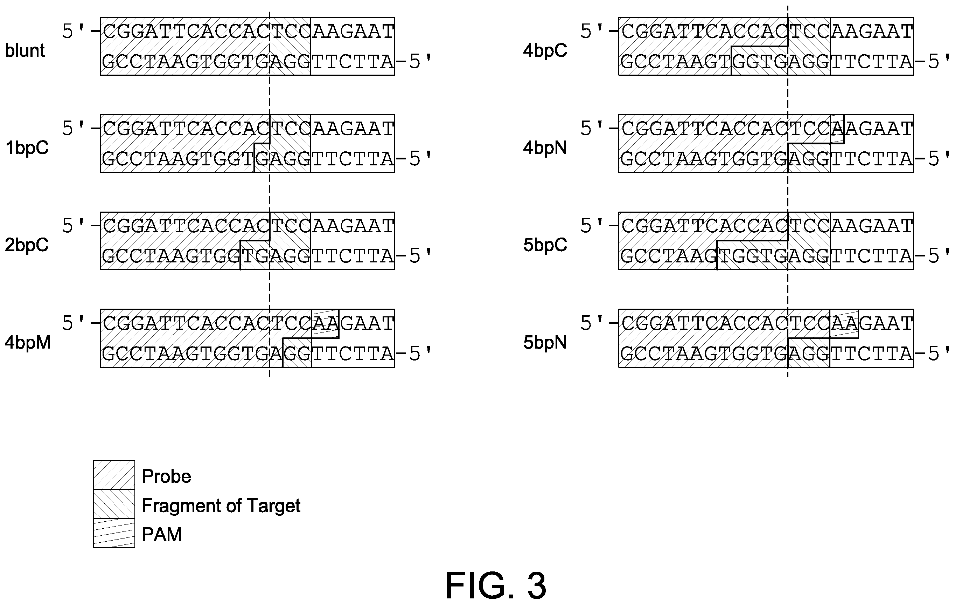

Various oligonucleotide capture probes were designed, as depicted in Figure 3. As shown in Figure 3, probes were designed with either blunt ends or cohesive ends with 3' overhangs. Figure 3 also depicts cleaved ends of various cleavage fragments of targets.

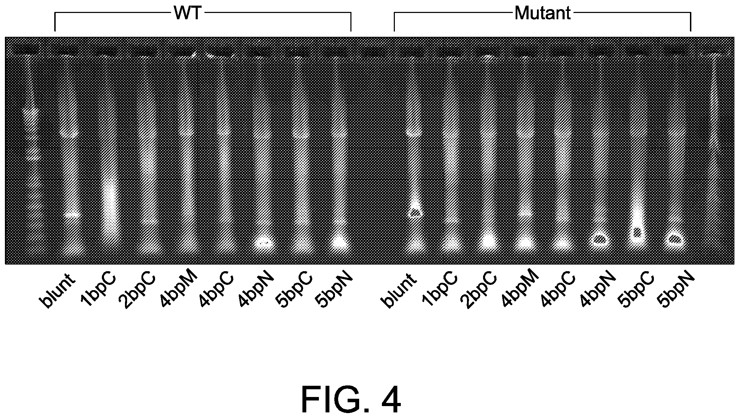

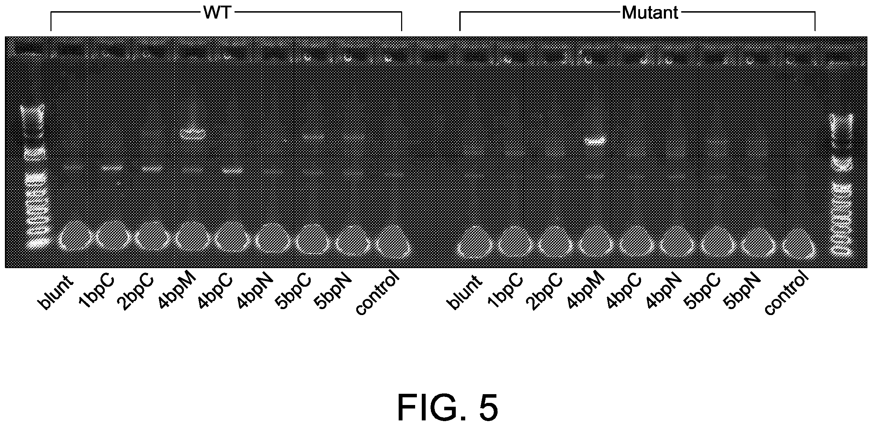

Wild type cas9 or libraries of cas9 variants were subjected to emulsified in vitro transcription and translation as described in Example 1. The reaction libraries were then purified using the DNA Clean & Concentrator™-5 product (Zymo Research) according to manufacturer's protocol. Purified libraries were then ligated to the variety of probes depicted in Figure 3, with either blunt ends ("blunt") or differing overhangs of lengths 1, 2, 4, and 5. Figure 4 shows the results using T4 ligase, while Figure 5 shows results using E. coli ligase. A control reaction was performed with no ligase and the maximum length of overhang. Successful ligation and amplification resulted in a band of about 4kb, seen at top in some reactions.

As shown in Figure 4, T4 ligase demonstrated low specificity for cleaved ends, and was able to successfully ligate oligonucleotide capture probes having blunt ends or cohesive ends to cleavage products. However, E. coli ligase was shown to be highly specific for cohesive ends (as shown in Figure 5).

**Equivalents**

It is to be understood that while the invention has been described in conjunction with the detailed description thereof, the foregoing description is intended to illustrate and not limit the scope of the invention, which is defined by the scope of the appended claims.

**Claims**

1.  A method comprising the steps of:

    (a) emulsifying a library comprising a plurality of nucleic acid templates to form a plurality of droplets, wherein each nucleic acid template comprises:

        (i) a first nucleotide sequence encoding a variant of an RNA-guided_nuclease operably linked to a first promoter; and
        (ii) a second nucleotide sequence comprising a target site for the RNA-guided nuclease; and

    wherein each of the droplets comprises a unique nucleic acid template;
    (b) expressing the RNA-guided nuclease variants in the plurality of droplets;
    (c) subjecting the plurality of droplets to conditions favorable for nuclease cleavage of the target site to produce a population of cleaved nucleic acid templates, each cleaved nucleic acid template comprising the first nucleotide sequence and a predetermined cleaved end; (d) ligating the cleaved nucleic acid templates with at least one oligonucleotide capture probe specific for the predetermined cleaved end to produce a plurality of ligation products; and

(e) identifying at least one first nucleotide sequence encoding an RNA-guided nuclease variant in at least one ligation product.

2. The method of claim 1, further comprising disrupting the droplets to obtain a mixture comprising cleaved nucleic acid templates, prior to the ligating step.

3. The method of claim 1, further comprising detecting, amplifying or sequencing at least one ligation product.

4. The method of claim 1, wherein each nucleic acid template further comprises a third nucleotide sequence encoding a guide RNA.

5. The method of claim 4, wherein the third nucleotide sequence is operably linked to the first promoter or to a second promoter.

6. The method of claim 1, wherein each nucleic acid template further comprises a fourth nucleotide sequence adjacent the target site, the fourth nucleotide sequence comprising a protospacer adjacent motif (PAM).

7. The method of claim 1, wherein the predetermined cleaved end comprises a 5' phosphate group, is a blunt end, or is a cohesive end.

8. The method of claim 7, wherein the cohesive end comprises a 3' overhang or a 5' overhang with a predetermined number of nucleotides.

9. The method of claim 1, wherein the ligating step comprises incubating with a T4 ligase or an E. coli ligase.

10. The method of claim 1, wherein step (d) comprises ligating the cleaved nucleic acid templates with a plurality of oligonucleotide capture probes, and optionally wherein each oligonucleotide capture probe is specific for a different predetermined cleaved end.

11. The method of claim 10, wherein each oligonucleotide capture probe comprises a unique detectable label associated with a predetermined cleaved end, optionally wherein the unique detectable label comprises a barcode sequence or a fluorescent marker.

12. The method of claim 1, wherein the step of emulsifying comprises forming an aqueous phase comprising the library of variant nucleic acid templates, and optionally wherein the emulsifying step further comprises adding the aqueous phase to a mixture comprising oil and surfactant to form a water-in-oil emulsion.

13. The method of claim 1, wherein the library comprises about $10^2$ to about $10^5$ variant nucleic acid templates.

14. A library comprising a plurality of variant nucleic acid templates encoding variants of a guide RNA, wherein each variant nucleic acid template comprises:

(i) a first nucleotide sequence encoding a nuclease operably linked to a first promoter;
(ii) a second nucleotide sequence comprising a detection sequence and encoding a variant of a guide RNA operably linked to a second promoter; and
(iii) a third nucleotide sequence comprising a target site for the guide RNA,

wherein upon expression of the nuclease and the guide RNA variants, the nuclease and one or more guide RNA variants form a nuclease/guide RNA variant complex that cleaves one or more nucleic acid templates producing a population of detectable cleaved nucleic acid templates comprising the second nucleotide sequence, wherein at least a portion of the detectable cleaved nucleic acid templates comprise a blunt end.

15. An emulsion comprising a plurality of droplets comprising a plurality of variant nucleic acid templates encoding variants of a guide RNA, wherein each of the droplets comprises a unique variant nucleic acid template comprising:

(i) a first nucleotide sequence encoding a nuclease operably linked to a first promoter;
(ii) a second nucleotide sequence comprising a detection sequence and encoding a variant of a guide RNA operably linked to a second promoter; and
(iii) a third nucleotide sequence comprising a target site for the guide RNA,

wherein upon expression of the nuclease and the guide RNA variants, the nuclease and one or more guide RNA variants form a nuclease/guide RNA variant complex that cleaves one or more nucleic acid templates producing a population of cleaved nucleic acid templates, each comprising the second nucleotide sequence and a predetermined cleaved end to which an oligonucleotide capture probe specific for the predetermined cleaved end can ligate.


**Patentansprüche**

1. Verfahren, umfassend die Schritte:

   (a) Emulgieren einer Bibliothek umfassend eine Vielzahl von Nukleinsäuretemplates zum Bilden einer Vielzahl von Tröpfchen,
   wobei jede Nukleinsäuretemplate umfasst:

   (i) eine erste Nukleotidsequenz codierend für eine Variante einer RNA-geführten Nuklease in Wirkverbindung mit einem ersten Promoter; und
   (ii) eine zweite Nukleotidsequenz umfassend eine Zielstelle für die RNA-geführte Nuklease; und

   wobei jedes der Tröpfchen eine eindeutige Nukleinsäuretemplate umfasst;
   (b) Exprimieren der RNA-geführten Nukleasevarianten in der Vielzahl von Tröpfchen;
   (c) Aussetzen der Vielzahl von Tröpfchen an Bedingungen, die eine Nukleasespaltung der Zielstelle begünstigen, um eine Population gespaltener Nukleinsäuretemplates herzustellen, wobei jede gespaltene Nukleinsäuretemplate die erste Nukleotidsequenz und ein vorbestimmtes gespaltenes Ende umfasst; (d) Ligieren der gespaltenen Nukleinsäuretemplates mit mindestens einer für das vorbestimmte gespaltene Ende spezifischen Oligonukleotid-Erfassungssonde, um eine Vielzahl von Ligationsprodukten herzustellen; und
   (e) Identifizieren mindestens einer ersten Nukleotidsequenz codierend für eine RNA-geführte Nukleasevariante in mindestens einem Ligationsprodukt.

2. Verfahren nach Anspruch 1, ferner umfassend Disruption der Tröpfchen zum Erhalten eines Gemischs umfassend gespaltene Nukleinsäuretemplates, vor dem Ligierungsschritt.

3. Verfahren nach Anspruch 1, ferner umfassend das Nachweisen, Amplifizieren oder Sequenzieren mindestens eines Ligationsprodukts.

4. Verfahren nach Anspruch 1, wobei jede Nukleinsäuretemplate ferner eine dritte Nukleotidsequenz, die für eine Führungs-RNA codiert, umfasst.

5. Verfahren nach Anspruch 4, wobei die dritte Nukleotidsequenz in Wirkverbindung mit dem ersten Promoter oder mit einem zweiten Promoter steht.

6. Verfahren nach Anspruch 1, wobei jede Nukleinsäuretemplate ferner eine vierte Nukleotidsequenz benachbart zu der Zielstelle umfasst, wobei die vierte Nukleotidsequenz ein Photospacer-Nachbarmotiv (Protospacer Adjacent Motif, PAM) umfasst.

7. Verfahren nach Anspruch 1, wobei das vorbestimmte gespaltene Ende eine 5'-Phosphatgruppe umfasst, ein stumpfes Ende ist oder ein kohäsives Ende ist.

8. Verfahren nach Anspruch 7, wobei das kohäsive Ende einen 3'-Überhang oder einen 5'-Überhang mit einer vorbestimmten Anzahl Nukleotide umfasst.

9. Verfahren nach Anspruch 1, wobei der Ligierungsschritt das Inkubieren mit einer T4-Ligase oder einer E.-coli-Ligase umfasst.

10. Verfahren nach Anspruch 1, wobei Schritt (d) das Ligieren der gespaltenen Nukleinsäuretemplates mit einer Vielzahl von Oligonukleotid-Erfassungssonden umfasst, und wahlweise, wobei jede Oligonukleotid-Erfassungssonde für ein unterschiedliches vorbestimmtes gespaltenes Ende spezifisch ist.

11. Verfahren nach Anspruch 10, wobei jede Oligonukleotid-Erfassungssonde eine eindeutige nachweisbare Markierung umfasst, die mit einem vorbestimmten gespaltenen Ende assoziiert ist, wahlweise, wobei die eindeutige nachweisbare Markierung eine Barcodesequenz oder einen Fluoreszenzmarker umfasst.

12. Verfahren nach Anspruch 1, wobei der Schritt des Emulgierens das Bilden einer wässrigen Phase umfassend die Bibliothek von varianten Nukleinsäuretemplates umfasst, und wahlweise, wobei der Emulgierungsschritt ferner das Hinzufügen der wässrigen Phase zu einem Gemisch umfassend Öl und Tensid zum Bilden einer Wasser-in-Öl-Emulsion umfasst.

13. Verfahren nach Anspruch 1, wobei die Bibliothek etwa $10^2$ bis etwa $10^5$ variante Nukleinsäuretemplates umfasst.

14. Bibliothek, umfassend eine Vielzahl von varianten Nukleinsäuretemplates, die für Varianten einer Führungs-RNA codieren, wobei jede variante Nukleinsäuretemplate umfasst:

(i) eine erste Nukleotidsequenz codierend für eine Nuklease in Wirkverbindung mit einem ersten Promoter;
(ii) eine zweite Nukleotidsequenz umfassend eine Nachweissequenz und codierend für eine Variante einer Führungs-RNA in Wirkverbindung mit einem zweiten Promoter; und
(iii) eine dritte Nukleotidsequenz umfassend eine Zielstelle für die Führungs-RNA,

wobei, bei Expression der Nuklease und der Führungs-RNA-Varianten, die Nuklease und eine oder mehrere Führungs-RNA-Varianten einen Nuklease/Führungs-RNA-Variantenkomplex bilden, der eine oder mehrere Nukleinsäuretemplates spaltet, wodurch eine Population nachweisbarer gespaltener Nukleinsäuretemplates umfassend die zweite Nukleotidsequenz hergestellt wird, wobei mindestens ein Abschnitt der nachweisbaren gespaltenen Nukleinsäuretemplates ein stumpfes Ende umfasst.

15. Emulsion, umfassend eine Vielzahl von Tröpfchen umfassend eine Vielzahl von varianten Nukleinsäuretemplates, die für Varianten einer Führungs-RNA codieren, wobei jedes der Tröpfchen eine eindeutige variante Nukleinsäuretemplate umfasst, umfassend:

(i) eine erste Nukleotidsequenz codierend für eine Nuklease in Wirkverbindung mit einem ersten Promoter;
(ii) eine zweite Nukleotidsequenz umfassend eine Nachweissequenz und codierend für eine Variante einer Führungs-RNA in Wirkverbindung mit einem zweiten Promoter; und
(iii) eine dritte Nukleotidsequenz umfassend eine Zielstelle für die Führungs-RNA,

wobei, bei Expression der Nuklease und der Führungs-RNA-Varianten, die Nuklease und eine oder mehrere Führungs-RNA-Varianten einen Nuklease/Führungs-RNA-Variantenkomplex bilden, der eine oder mehrere Nukleinsäuretemplates spaltet, wodurch eine Population gespaltener Nukleinsäuretemplates hergestellt wird, jeweils umfassend die zweite Nukleotidsequenz und ein vorbestimmtes gespaltenes Ende, woran eine für das vorbestimmte gespaltene Ende spezifische Oligonukleotid-Erfassungssonde ligieren kann.

**Revendications**

1. Procédé comprenant les étapes consistant à :

(a) émulsionner une collection comprenant une pluralité de variantes de matrices d'acide nucléique pour former une pluralité de gouttelettes,
chaque matrice d'acide nucléique comprenant :

(i) une première séquence de nucléotides codant une variante d'une nucléase guidée par ARN liée de manière fonctionnelle à un premier promoteur ; et
(ii) une deuxième séquence de nucléotides comprenant un site cible pour la nucléase guidée par ARN ; et

chacune des gouttelettes comprenant une unique matrice d'acide nucléique ;
(b) exprimer les variantes de nucléase guidée par ARN dans la pluralité de gouttelettes ;
(c) soumettre la pluralité de gouttelettes à des conditions favorables à un clivage par nucléase du site cible afin de produire une population de matrices d'acide nucléique clivées, chaque matrice d'acide nucléique clivée comprenant la première séquence de nucléotides et une extrémité clivée prédéterminée ;

(d) ligaturer les matrices d'acide nucléique clivées avec au moins une sonde de capture oligonucléotidique spécifique de l'extrémité clivée prédéterminée afin de produire une pluralité de produits de ligature ; et

(e) identifier au moins une première séquence de nucléotides codant une variante de nucléase guidée par ARN dans au moins un produit de ligature.

2. Procédé selon la revendication 1, consistant en outre à interrompre les gouttelettes afin d'obtenir un mélange comprenant des matrices d'acide nucléique clivées avant l'étape de ligature.

3. Procédé selon la revendication 1, consistant en outre à détecter, amplifier ou séquencer au moins un produit de ligature.

4. Procédé selon la revendication 1, dans lequel chaque matrice d'acide nucléique comprend en outre une troisième séquence de nucléotides codant un ARN guide.

5. Procédé selon la revendication 4, dans lequel la troisième séquence de nucléotides est liée de manière fonctionnelle au premier promoteur ou à un second promoteur.

6. Procédé selon la revendication 1, dans lequel chaque matrice d'acide nucléique comprend en outre une quatrième séquence de nucléotides adjacente au site cible, la quatrième séquence de nucléotides comprenant un motif adjacent de protoespaceur (PAM).

7. Procédé selon la revendication 1, dans lequel l'extrémité clivée prédéterminée comprend un groupe phosphate en 5', est une extrémité franche ou une extrémité cohésive.

8. Procédé selon la revendication 7, dans lequel l'extrémité cohésive comprend une extrémité sortante 3' ou une extrémité sortante 5' avec un nombre prédéterminé de nucléotides.

9. Procédé selon la revendication 1, dans lequel l'étape de ligature consiste à réaliser une incubation avec une ligase T4 ou une ligase d'E. coli.

10. Procédé selon la revendication 1, dans lequel l'étape (d) consiste à ligaturer les matrices d'acide nucléique clivées avec une pluralité de sondes de capture oligonucléotidiques, et éventuellement dans lequel chaque sonde de capture oligonucléotidique est spécifique d'une extrémité clivée prédéterminée différente.

11. Procédé selon la revendication 10, dans lequel chaque sonde de capture oligonucléotidique comprend un unique marqueur détectable associé à une extrémité clivée prédéterminée, et éventuellement dans lequel l'unique marqueur détectable comprend une séquence de codes-barres ou un marqueur fluorescent.

12. Procédé selon la revendication 1, dans lequel l'étape d'émulsion consiste à former une phase aqueuse comprenant la collection de variantes de matrices d'acide nucléique, et éventuellement dans lequel l'étape d'émulsion consiste en outre à ajouter la phase aqueuse à un mélange comprenant de l'huile et un agent tensio-actif pour former une émulsion eau dans huile.

13. Procédé selon la revendication 1, dans lequel la collection comprend environ $10^2$ à environ $10^5$ variantes de matrices d'acide nucléique.

14. Collection comprenant une pluralité de variantes de matrices d'acide nucléique d'un ARN guide, chaque variante de matrice d'acide nucléique comprenant :

(i) une première séquence de nucléotides codant une nucléase liée de manière fonctionnelle à un premier promoteur ;

(ii) une deuxième séquence de nucléotides comprenant une séquence de détection et codant une variante d'un ARN guide lié de manière fonctionnelle à un second promoteur ; et

(iii) une troisième séquence de nucléotides comprenant un site cible pour l'ARN guide,

au moment de l'expression de la nucléase et des variantes d'ARN guide, la nucléase et une ou plusieurs variantes d'ARN guide formant un complexe nucléase/variante d'ARN guide qui clive une ou plusieurs matrices d'acide nucléique produisant une population de matrices d'acide nucléique clivées détectables comprenant la deuxième séquence de nucléotides, au moins une partie des matrices d'acide nucléique clivées détectables comprenant une extrémité franche.

15. Émulsion comprenant une pluralité de gouttelettes comprenant une pluralité de variantes de matrices d'acide nucléique codant des variantes d'un ARN guide, chacune des gouttelettes comprenant une variante de matrice d'acide nucléique unique, comprenant :

(i) une première séquence de nucléotides codant une nucléase liée de manière fonctionnelle à un premier promoteur ;
(ii) une deuxième séquence de nucléotides comprenant une séquence de détection et codant une variante d'un ARN guide lié de manière fonctionnelle à un second promoteur ; et
(iii) une troisième séquence de nucléotides comprenant un site cible pour l'ARN guide,

au moment de l'expression de la nucléase et des variantes d'ARN guide, la nucléase et une ou plusieurs variantes d'ARN guide formant un complexe nucléase/variante d'ARN guide qui clive une ou plusieurs matrices d'acide nucléique produisant une population de matrices d'acide nucléique clivées, comprenant chacune la deuxième séquence de nucléotides et une extrémité clivée prédéterminée à laquelle une sonde de capture oligonucléotidique spécifique de l'extrémité clivée prédéterminée peut se ligaturer.

| Target site | First promoter | Cas9 | Second promoter | gRNA | Terminator |

# FIG. 1

Library generation

Emulsified in vitro transcription and translation

5'  3'

5'P  3'

Probe capture

5'P  3'

Ligase

PCR enrichment

Deep sequencing Validation

FIG. 2

EP 3 555 288 B1

FIG. 3

EP 3 555 288 B1

FIG. 4

FIG. 5

REFERENCES CITED IN THE DESCRIPTION

**Patent documents cited in the description**

- WO 9902671 A
- US 20080004436 A
- WO 2016073990 A

**Non-patent literature cited in the description**

- *U.S. Food and Drug Administration (FDA) held a Science Board Meeting,* 15 November 2016
- **MILLER et al.** *Nature Methods,* 2006, vol. 3, 561-570
- **ZUBAY.** *Annu Rev Genet.,* 1973, vol. 7, 267-287
- **LESLEY et al.** *J Biol. Chem.,* vol. 266 (4), 2632-2638
- **PELHAM ; JACKSON.** *Eur J. Biochem.,* 1976, vol. 67 (1), 247-256
- **SHMAKOV et al.** *Molecular Cell,* 05 November 2015, vol. 60, 385-397
- **RAN ; HSU et al.** *Cell,* 12 September 2013, vol. 154 (6), 1380-1389
- **JINEK et al.** *Science,* 2014, vol. 343 (6176), 1247997
- **ANDERS et al.** *Nature,* 25 September 2014, vol. 513 (7519), 569-73
- **YAMANO et al.** *Cell,* 05 May 2016, vol. 165 (4), 949-962
- **CONG et al.** *Science,* 15 February 2013, vol. 339 (6121), 819-23
- **WANG et al.** *PLoS One,* 31 December 2013, vol. 8 (12), e85650
- **BRINER et al.** *Molecular Cell,* 23 October 2014, vol. 56 (2), 333-339
- **MALI et al.** *Science,* 15 February 2013, vol. 339 (6121), 823-826
- **JIANG et al.** *Nat Biotechnol.,* March 2013, vol. 31 (3
- **JINEK et al.** *Science,* 17 August 2012, vol. 337 (6096), 816-821
- **HSU et al.** *Nat Biotechnol.,* September 2013, vol. 31 (9), 827-832
- **NISHIMASU et al.** *Cell,* 27 February 2014, vol. 156, 935-949
- **NISHIMASU et al.** *Cell,* 27 August 2015, vol. 162, 1113-1126
- **ZETSCHE et al.** *Cell,* 22 October 2015, vol. 163, 759-771